# FASCICULE DE BREVET EUROPEEN

(11) **EP 1 341 753 B1**
(45) Date de publication et mention de la délivrance du brevet: **26.09.2007**
(21) Numéro de dépôt: 01998533.2
(22) Date de dépôt: 30.11.2001
(51) Int. Cl.: C07C 311/08, A61K 31/195

(54) **CYCLOHEXYL(ALKYL)-PROPANOLAMINES, LEUR PREPARATION ET COMPOSITIONS PHARMACEUTIQUES EN CONTENANT**
CYCLOHEXYL(ALKYL)-PROPANOLAMINE, VERFAHREN ZU IHRER HERSTELLUNG UND DIESE ENTHALTENDE PHARMAZEUTISCHE ZUSAMMENSETZUNGEN
CYCLOHEXYL(ALKYL)-PROPANOLAMINES, PREPARATION METHOD AND PHARMACEUTICAL COMPOSITIONS CONTAINING SAME

(30) Priorité: 30.11.2000 FR 0015477
(43) Date de publication de la demande: 10.09.2003
(73) Titulaire: Sanofi-Aventis, 75013 Paris (FR)
(72) Inventeur: BOVY, Philippe, R., F-78750 Mareil Marly (FR); CECCHI, Roberto, I-26900 Lodi (IT); COURTEMANCHE, Gilles, 92350 Le Plessis Robinson (FR); OLIVA, Ambrogio, I-21047 Sarrono (IT); VIVIANI, Nunzia, It-22063 Cantu' (IT); CROCI, Tiziano, I-20156 Milano (IT)
(74) Mandataire: Varady, Peter
(86) Numéro de dépôt international: PCT/FR2001/003784
(87) Numéro de publication internationale: WO 2002/044139

(56) Documents cités:
- EP-A- 0 345 056
- WO-A-99/65895
- US-A- 4 004 028

## Description

La présente invention concerne de nouvelles cyclohexyl(alkyl)-propanolamines, les compositions pharmaceutiques les contenant, un procédé pour leur préparation et des intermédiaires de synthèse dans ce procédé.

WO99/65895 décrit des phénoxypropanolamines dans lesquelles l'amine porte une pipéridine substituée, ces composés montrant une activité agoniste vis-à-vis du récepteur bêta-3 adrénergique.

Le récepteur bêta-3 adrénergique a fait l'objet de nombreuses études visant à synthétiser des composés agonistes vis-à-vis de ce récepteur, ces composés exerçant un effet anti-obésité et anti-diabétique important chez l'homme, comme décrit par exemple par Weyer, C et al., Diabetes Metab., 1999, 25(1) :11-21.

Il a été maintenant trouvé que certaines propanolamines portant un groupe cyclohexyl(alkyl)e sur l'amine possèdent une puissante activité agoniste vis-à-vis des récepteurs bêta-3 adrénergiques.

Ainsi, la présente invention concerne, selon un de ses aspects, des cyclohexyl(alkyl)-propanolamines de formule (I) : 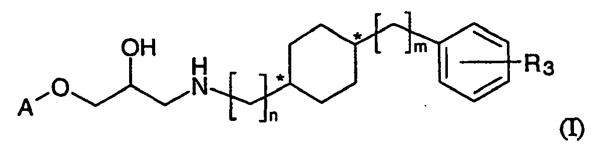 dans laquelle
A est un groupe de formule (a) ou (b) 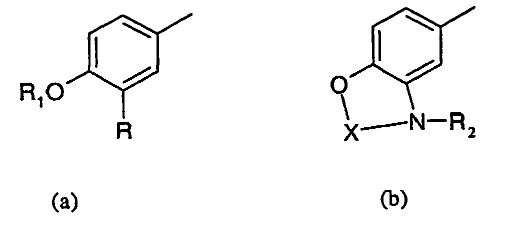
où
- R: représente un atome d'hydrogène ou d'halogène, un groupe -S(O)₂(C₁-C₄)alkyle, un groupe -NHSO₂(C₁-C₄)alkyle, un groupe -SO₂NH(C₁-C₄)alkyle, un groupe -NHSO₂phényl-(C₁-C₄)alkyle ou un groupe -NHSO₂phényle, ledit phényle pouvant être substitué par un atome d'halogène, par un groupe (C₁-C₄)alkyle ou par un groupe (C₁-C₄)alcoxy ;
- R₁: représente un atome d'hydrogène ou un groupe (C₁-C₄)alkyle, un groupe -CO(C₁-C₄)alkyle, un groupe phényl-(C₁-C₄)alkyle ou un groupe -CO-phényle, ledit phényle pouvant être substitué par un atome d'halogène ou par un groupe (C₁-C₄)alcoxy;
- R₂: est un atome d'hydrogène, un groupe -SO₂(C₁-C₄)alkyle, un groupe -SO₂phényl-(C₁-C₄)alkyle ou un groupe -SO₂phényle ;
- X: complète un cycle de 5 à 8 atomes, ledit cycle étant saturé ou insaturé, pouvant être substitué par un ou deux groupes (C₁-C₄)alkyles et portant un ou deux groupes carbonyles ;
- n, m et z: sont indépendamment 0, 1 ou 2 ;
- R₃: représente un atome d'hydrogène ou d'halogène, un groupe (C₁-C₆)alkyle, un groupe (C₁-C₄)alcoxy, un groupe -COO(C₁-C₄)alkyle, un groupe -CO-(C₁-C₄)alkyle, un groupe -NHSO₂(C₁-C₄)alkyle, un groupe NHSO₂phényl-(C₁-C₄)alkyle, -NO₂, -CN, -CONR₄R₅, -COOH, un groupe 4,5-dihydro-1,3-oxazol-2-yle ou 4,4-diméthyl-4,5-dihydro-1,3-oxazol-2-yle ;
- R₄ et R₅: représentent indépendamment un atome d'hydrogène, un phényle, un groupe (C₁-C₄)alkyle ou un groupe phényl-(C₁-C₄)alkyle ;
ou bien
- R₄ et R₅: avec l'atome d'azote auquel ils sont liés, peuvent former un cycle de 5 à 7 atomes au total ;
ainsi que leurs sels ou solvates.

Dans la présente description, le terme "(C₁-C₄)alkyle" et "(C₁-C₆)alkyle" désignent des radicaux monovalents d'un hydrocarbure respectivement en C₁-C₄ et C₁-C₆ à chaîne saturée droite ou ramifiée.

Dans la présente description, le terme "halogène" désigne un atome choisi parmi le chlore, le brome, le iode et le fluor.

Des composés préférés sont ceux où n et m sont chacun zéro.

D'autres composés préférés sont ceux où R₁ est un atome d'hydrogène.

D'autres composés préférés sont ceux où R est choisi parmi un groupe -NHSO₂(C₁-C₄)alkyle, un groupe -NHSO₂phényl-(C₁-C₄)alkyle ou un groupe -NHSO₂phényle.

D'autres composés préférés sont ceux où R₃ est -COO(C₁-C₄)alkyle ou -CO(C₁-C₄)alkyle ou CONR₄R₅.

D'autres composés préférés sont ceux où R₄ est dans la position 4 du benzène.

D'autres composés préférés sont ceux où z est 2.

D'autres composés préférés sont ceux où X est un méthylène, un éthylène ou un propylène.

D'autres composés préférés sont ceux où X est un carbonyle, un groupe-COCO-, un groupe -CO-C((C₁-C₄)alkyle)₂-CO-, un méthylène mono- ou di-substitué par (C₁-C₄)alkyle ou un groupe -COCH₂-.

Des groupes -NHSO₂phényl-(C₁-C₄)alkyle et -SO₂phényl-(C₁-C₄)alkyle préférés sont respectivement le benzylsulfonyl-amino et le benzylsulfonyle.

Lorsque R₄ et R₅ forment avec l'atome d'azote auquel ils sont liés, un cycle de 5 à 7 atomes, des cycles préférés sont la pipéridine et la pyrrolidine.

Les sels des composés de formule (I) selon la présente invention comprennent aussi bien les sels d'addition avec des acides minéraux ou organiques pharmaceutiquement acceptables tels que le chlorhydrate, le bromhydrate, le sulfate, l'hydrogénosulfate, le dihydrogénophosphate, le citrate, le maléate, le tartrate, le fumarate, le gluconate, le méthanesulfonate, le 2-naphtalènesulfonate, etc., que les sels d'addition qui permettent une séparation ou une cristallisation convenable des composés de formule (I), tels que le picrate, l'oxalate ou les sels d'addition avec des acides optiquement actifs, par exemple les acides camphosulfoniques et les acides mandéliques ou mandéliques substitués.

Lorsque les composés de formule (I) possèdent un groupe carboxy libre les sels comprennent aussi les sels avec des bases minérales, de préférence celles avec des métaux alcalins tels que le sodium ou le potassium, ou avec des bases organiques.

Les stéréoisomères optiquement purs, ainsi que les mélanges d'isomères des composés de formule (I), dus au carbone asymétrique, dans une proportion quelconque, font partie de la présente invention.

Des composés de formule (I) préférés sont les composés où la configuration du carbone de la propanolamine portant le groupe OH est (S).

Les composés de formule (I) peuvent se présenter sous le forme d'isomères géométriques « cis » ou « trans » selon la position relative des substituants dans les position 1 et 4 du noyau cyclohexyle (marqués par une étoile). Ces isomères purs et leurs mélanges, dans une proportion quelconque, font partie de la présente invention.

Les mélanges de stéréoisomères optiques et géométriques ci-dessus, dans une proportion quelconque, sont également partie de la présente invention.

Les composés de formule (I) peuvent être préparés en traitant un composé de formule (II) : 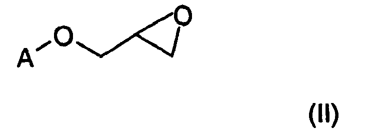 dans laquelle A est tel qu'indiqué ci-dessus, avec une amine de formule (III) 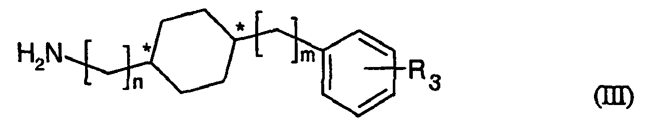 où n, m et R₃ sont tels que définis ci-dessus et éventuellement en transformant le composé de formule (I) ainsi obtenu en un de ses sels ou solvates.

Plus particulièrement, la réaction entre les composés de formule (II) et (III) est réalisée dans un solvant organique, tel qu'un alcool inférieur comme le méthanol, l'éthanol, l'isopropanol et le tert-butanol; le diméthylsulfoxyde; un éther linéaire ou cyclique; un amide comme le diméthylformamide ou le diméthylacétamide ou encore des mélanges de ces solvants; en utilisant préférablement des quantités au moins équimoléculaires des réactifs.

La température de la réaction est comprise entre la température ambiante et la température de reflux du solvant choisi.

Lorsque R₁ représente l'hydrogène, il est préférable de protéger le groupe fonctionnel par un groupe protecteur afin d'augmenter le rendement de la réaction. Comme groupes protecteurs, on peut utiliser les groupes protecteurs usuels pour les groupes phénols tels que par exemple le méthoxyéthoxyméthyle (MEM) le triméthylsilyl-éthoxyméthyle (SEM), le benzyle, éventuellement substitué, ou le benzoyle, selon les techniques bien connues.

D'autres groupes fonctionnels éventuellement présents (les groupes amino par exemple) peuvent eux aussi être protégés par des groupes protecteurs appropriés selon les techniques conventionnelles bien connues.

Les composés de formule (II) sont des composés connus en littérature ou bien ils peuvent être préparés par des procédés analogues aux procédés décrits.

Les composés de formule (III) peuvent être préparés à partir d'un intermédiaire de formule (IX) obtenu selon le schéma 1 suivant. 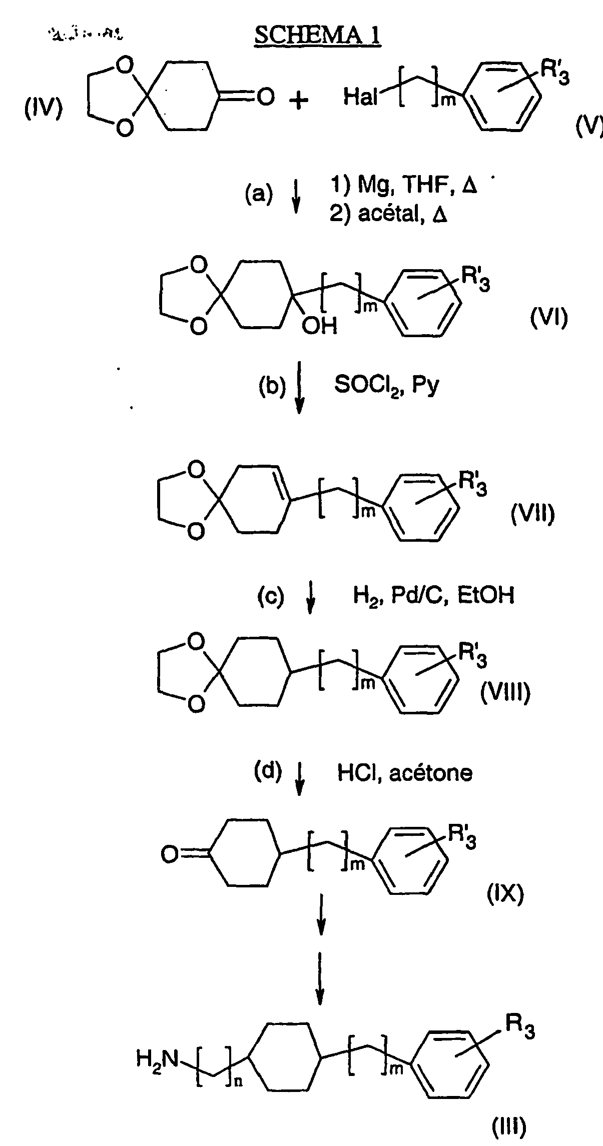 Dans les formules du Schéma 1, m est tel que défini ci-dessus et Hal représente un halogène, de préférence le brome, alors que R'₃ est un groupe 4,5-dihydro-1,3-oxazol-2-yle, 4,4-diméthyl-4,5-dihydro-1,3-oxazol-2-yle, (C₁-C₄)alkyle ou (C₁-C₄)alcoxy.

Il s'agit de réactions bien connues à l'homme de métier; l'étape de condensation peut être par exemple conduite d'une façon analogue à celle décrite par Meyers et al., J. Org. Chem., 1974, 39 :2787. L'alcol intermédiaire (VI) est transformé dans le dérivé (VII) par exemple selon la méthode décrite dans A.M. Gonzales-Cameno et al. Tetrahedron, 1994, 50 :10971 ou bien avec POCl₃ comme décrit dans Org. Prep. Proced. Int., 1995, 27 :122, et ensuite dans le dérivé saturé (VIII) par une réaction de réduction conventionnelle. L'hydrolyse du groupe acétal peut être réalisée de façon analogue à la réaction décrite par C. Szantay et al., Tetrahedron, 1996, 52(33) :11053.

L'intermédiaire de formule (IX) peut être utilisé pour préparer les composés de formule (III) en transformant par exemple le groupe carbonyle du cyclohexane en groupe amino par réduction de l'oxime correspondant ou lorsque n est 1 ou 2, en groupe aminoalkyle par réaction avec un cyanure o nitrométhane, ou par la réaction de Wittig avec le phosphonate souhaité, selon des réactions bien connues.

Lorsque R'₃ est un groupe 4,5-dihydro-1,3-oxazol-2-yle ou 4,4-diméthyl-4,5-dihydro-1,3-oxazol-2-yle le composé de formule (III) où R₃ est -COOH est aisément obtenu par hydrolyse.

La plupart des composés de formule (III), tels que par exemple ceux où R₃ est -CN, -CO(C₁-C₄)alkyle, -COO(C₁-C₄)alkyle, un groupe sulfonamido, un atome d'halogène ou -NO₂ peuvent après être obtenus par transformation du composé de formule (III) où R₃ est -COOH, selon des réactions bien connues à l'homme du métier après protection du groupe NH₂ par un groupe protecteur comme par exemple le groupe BOC et/ou le benzyle.

Alternativement, on peut utiliser comme produit de départ le composé de formule (V) où R'₃ est -CN, et m=0, 1; dans ce cas, la condensation de l'étape (a) est conduite comme décrit par exemple dans J. Med. Chem., 1992, 35 :320 et le produit (III) obtenu à la fin du procédé ci-dessus est aisément transformé en les dérivés où R₃ est -COOH, -CO(C₁-C₄)alkyle ou -CONR₄R₅ après protection du groupe NH₂ par un groupe protecteur comme par exemple le groupe BOC (*tert*-butoxycarbonyle) et/ou le benzyle.

Les isomères « cis » et « trans » peuvent être obtenus par séparation du mélange, par exemple par chromatographie ou par cristallisation sélective, selon des procédés conventionnels.

L'activité des composés de la présente invention vis-à-vis de l'activité bêta-3 a été mise en évidence à l'aide d'essais in vitro sur le colon humain selon la méthode décrite par T. Croci et al, Br. J. Pharmacol., 1997, 122: 139P par L. Manara et al., Gut, 2000, 47:337-342 et dans EP-B-436435.

Plus particulièrement, on a constaté que les composés de formule (I) sont beaucoup plus actifs sur le côlon isolé que sur l'oreillette et sur la trachée.

Ces propriétés surprenantes des composés de formule (I) permettent d'envisager leur utilisation comme médicaments à action bêta-3 agoniste.

De plus, les composés de formule (I) sont peu toxiques; notamment, leur toxicité aiguë est compatible avec leur utilisation comme médicaments pour le traitement de maladies dans lesquelles les composés ayant une affinité pour le récepteur bêta-3, notamment les bêta-3 agonistes, trouvent leur application. De telles maladies sont décrites en littérature. Les composés de formule (I), ainsi que leurs sels pharmaceutiquement acceptables, peuvent donc par exemple être indiqués dans le traitement des maladies gastro-intestinales telles que les maladies inflammatoires de intestin telles que le syndrôme du colon irritable (IBD), comme modulateurs de la motricité intestinale, comme lipolytiques, agents anti-obésité, anti-diabétiques, psychotropes, anti-glaucomateux, cicatrisants, anti-dépresseurs, comme inhibiteur des contractions utérines, comme tocolytiques pour prévenir ou retarder les accouchement précoces, pour le traitement et/ou la prophylaxie de la dysménorrhée. En outre les composés de formule (I) peuvent être utilisés dans le traitement de certaines maladies du système nerveux central, telle que par exemple la dépression, ainsi que de certains troubles du système urinaire tel que l'incontinence urinaire.

L'utilisation des composés de formule (I) ci-dessus, ainsi que celle de leurs sels et solvates pharmaceutiquement acceptables pour la préparation de médicaments ci-dessus, constitue un aspect ultérieur de la présente invention.

Pour une telle utilisation, on administre aux mammifères qui nécessitent un tel traitement une quantité efficace d'un composé de formule (I) ou d'un de ses sels et solvates pharmaceutiquement acceptables.

Les composés de formule (I) ci-dessus et leurs sels et solvates pharmaceutiquement acceptables peuvent être utilisés à des doses journalières de 0,01 à 20 mg par kilo de poids corporel du mammifère à traiter, de préférence à des doses journalières de 0,1 à 10 mg/kg. Chez l'être humain, la dose peut varier de préférence de 0,5 mg à 1500 mg par jour, notamment de 2,5 à 500 mg selon l'âge du sujet à traiter, le type de traitement, prophylactique ou curatif, et la gravité de l'affection. Les composés de formule (I) sont généralement administrés en unité de dosage de 0,1 à 500 mg, de préférence de 0,5 à 100 mg de principe actif, une à cinq fois par jour.

Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ci-dessus ou un de ses sels et solvates pharmaceutiquement acceptables.

Dans les compositions pharmaceutiques de la présente invention pour l'administration orale, sublinguale, sous-cutanée, intramusculaire, intraveineuse, topique, transdermique ou rectale, les ingrédients actifs de formule (I) ci-dessus, leurs sels et solvates pharmaceutiquement acceptables peuvent être administrés sous formes unitaires d'administration, en mélange avec des supports pharmaceutiques classiques, aux animaux et aux êtres humains, pour le traitement des affections susdites. Les formes unitaires d'administration appropriées comprennent les formes par voie orale telles que les comprimés, les gélules, les poudres, les granulés et les solutions ou suspensions orales, les formes d'administration sublinguale et buccale, les formes d'administration sous-cutanée, intramusculaire ou intraveineuse, les formes d'administration locale et les formes d'administration rectale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granulés dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration locale, on mélange le principe actif dans un excipient pour la préparation de crèmes ou onguents ou on le dissout dans un véhicule pour l'administration intraoculaire, par exemple sous forme de collyre.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Selon un autre de ses aspects, la présente invention concerne une méthode de traitement des pathologies qui sont améliorées par une action bêta-3-agoniste, qui comprend administrer un composé de formule (I) ou l'un de ses sels ou solvates pharmaceutiquement acceptables.

Les composés de formule (I) notamment les composés (I) marquées par un isotope, peuvent aussi être utilisés comme outils de laboratoire dans des essais biochimiques.

Les composés de formule (I) se lient au récepteur bêta-3-adrénergique. On peut donc utiliser ces composés dans un essai ordinaire de liaison ("binding"), dans lequel on emploie un tissu organique où ce récepteur est particulièrement abondant, et on mesure la quantité de composé (I) déplacé par un composé test, pour évaluer l'affinité dudit composé vis-à-vis des sites de liaison de ce récepteur particulier.

Un autre objet spécifique de la présente invention est donc un réactif utilisable dans les essais biochimiques, qui comprend au moins un composé de formule (I) convenablement marqué.

Les exemples qui suivent illustrent mieux l'invention. Dans ces exemples les abréviations suivantes pourront être utilisées:
Ph = phényle; Bn = benzyle; Me= méthyle; Et = éthyle; Bu = butyle; Ox = 4,4-diméthyl-4,5-dihydro-1,3-oxazol-2-yle.

### PREPARATION 1

### 4-(4-oxocyclohexyl)-benzoate d'éthyle

### (i) 8-[4-(4,4-diméthyl-4,5-dihydro-1,3-oxazol-2-yl)-phényl]-1,4-dioxaspiro[4,5]decan-8-ol

On verse goutte à goutte une solution de 8 g de 2-(4-bromophényl)-4,4-diméthyl-4,5-dihydro-1,3-oxazole (31,5 mmole) dans 15 ml de THF anhydre sur 956 mg de Mg (39,3 mmole) de façon à maintenir le mélange au reflux et on chauffe le mélange au reflux pendant 2,5 heures. On refroidit le mélange à la température ambiante, et on y ajoute goutte à goutte une solution de 5,41 g de 1,4-cyclohexandione monoéthylèneacétal (34,65 mmole) dans 20 ml de THF anhydre. On agite le mélange à la température ambiante pendant 1,5 heures et après au reflux pendant 1 heure. On verse le mélange dans 500 ml d'une solution de NH₄Cl à 10% et on extrait à l'acétate d'éthyle. On sèche la phase organique, on filtre et on évapore le solvant sous pression réduite. On cristallise le résidu dans de l'acétate d'éthyle. On obtient le produit du titre sous forme de solide blanc.
P.f. 146-148°C.

### (ii) 2-[4-(1,4-dioxaspiro[4,5]dec-7-èn-8-yl)-phényl]-4,4-diméthyl-4,5-dihydro-1,3-oxazole

A une solution de 34 g du produit de l'étape précédente (102,6 mmole) dans 250 ml de chlorure de méthylène on ajoute 73,5 ml de pyridine ; on refroidit le mélange à 0°C et on y ajoute goutte à goutte 15 ml de chlorure de thionyle (205 mmole) pendant 15 minutes. Après une heure on enlève le bain de refroidissement et après une heure on chauffe au reflux pendant une heure. On évapore le solvant et on ajoute 400 ml d'eau et 250 ml d'acétate d'éthyle. On lave la phase organique avec une solution aqueuse saturée en NaCl. On extrait la phase aqueuse avec de l'acétate d'éthyle, on sèche les phase organiques réunies, on filtre et on évapore le solvant sous pression réduite. On obtient le produit du titre sous forme de solide blanc qu'on cristallise dans un mélange acétate d'éthyle/hexane.
P.f. 109-111°C.

### (iii) 2-[4-(1,4-dioxaspiro[4,5]dec-8-yl)-phényl]-4,4-diméthyl-4,5-dihydro-1,3-oxazole

On hydrogène une solution de 33,1 g du produit de l'étape précédente (105,6 mmole) dans 350 ml d'éthanol absolu en présence de 4,0 g de Pd/C à 10%, à la pression de 1 atmosphère et à 30°C, pendant 5 heures. On filtre le catalyseur et on évapore le solvant. On reprend le résidu dans de l'hexane et on filtre un solide blanc. On obtient ainsi le composé du titre.
P.f. 128-140°C.

### (iv) 4-(4-oxocyclohexyl)-benzoate d'éthyle

On dissous 3 g du produit de l'étape (iii) (9,51 mmole) dans 60 ml d'éthanol et on ajoute 4,0 ml d'acide sulfurique à 96% et on chauffe le mélange au reflux pendant 22 heures. On évapore partiellement le solvant et on reprend avec un mélange de 300 ml d'une solution saturée en bicarbonate de sodium et 150 ml d'acétate d'éthyle. On lave la phase organique avec une solution aqueuse saturée en NaCl. On sèche la phase organique, on filtre et on évapore le solvant sous pression réduite. On purifie le produit brut par chromatographie sur colonne de gel de silice en éluant avec un mélange hexane/acétate d'éthyle=8/2. On obtient le composé du titre sous forme de solide.
P.f. 60-62°C.

### PREPARATION 2

### 4-[4-(4,4-diméthyl-4,5-dihydro-1,3-oxazol-2-yl)-phényl]-cyclohexanone

On dissous 2 g du produit de l'étape (iii) de la Préparation 1 (6,34 mmole) dans 70 ml d'acétone et on y ajoute 4 ml d'acide chlorhydrique 6N. On agite le mélange à la température ambiante pendant 20 heures. On évapore le solvant et on reprend avec un mélange de 250 ml d'une solution de bicarbonate de sodium à 5% et 200 ml d'acétate d'éthyle. On lave la phase organique à l'eau et avec une solution aqueuse saturée en NaCl. On sèche la phase organique, on filtre et on évapore le solvant sous pression réduite. On purifie le produit brut par chromatographie sur colonne de gel de silice en éluant avec un mélange hexane/acétate d'éthyle=6/4. On obtient le composé du titre sous forme de solide blanc.
P.f. 118-120°C.

### PREPARATION 3

### 4-(4-aminocyclohexyl)-benzoate d'éthyle (cis et trans) et chlorhydrate de l'isomère trans.

### (i) 4-[4-(methoxyimino)-cyclohexyl]-benzoate d'éthyle

On dissout 2,88 g du produit de la Préparation 1 (11,7 mmole) dans 25 ml d'éthanol et on y ajoute 1,17 g de O-methyl-hydroxylamine (14 mmole) et 5 ml de pyridine. On agite le mélange à 50°C pendant 4 heures. On évapore le solvant et on reprend avec un mélange de 50 ml d'eau et 50 ml d'acétate d'éthyle. On lave la phase organique à l'eau et avec une solution aqueuse saturée en NaCl. On sèche la phase organique, on filtre et on évapore le solvant sous pression réduite. On purifie le produit brut par chromatographie sur colonne de gel de silice en éluant avec un mélange hexane/acétate d'éthyle=90/10. On obtient le composé du titre sous forme de solide blanc.
P.f. 74-76°C.

### (ii) 4-(4-aminocyclohexyl)-benzoate d'éthyle (cis et trans) et chlorhydrate de l'isomère trans

On dissout 1,42 g du produit de l'étape précédente (5,16 mmole) dans 5 ml de THF, en atmosphère d'azote à 0°C et on ajoute pendant 10 minutes 11,3 ml une solution 1 M de hydrure de bore dans du THF (11,3 mmole). On agite le mélange pendant 5h à la température ambiante et après au reflux pendant une heure. On ajoute au mélange 30 ml d'éthanol et on agite pendant 1 heure au reflux ensuite on y ajoute 3 ml d'éthanol saturé en acide chlorhydrique et on agite a 70°C pendant 2 heures. On évapore le solvant et on reprend avec un mélange de 40 ml d'une solution saturée en bicarbonate de sodium et 40 ml d'acétate d'éthyle. On lave la phase organique avec une solution aqueuse saturée en NaCl. On sèche le phase organique, on filtre et on évapore le solvant sous pression réduite. On purifie le produit brut par chromatographie sur colonne de gel de silice en éluant avec un mélange chlorure de méthylène/méthanol/NH₄OH=94/6/0,6. On obtient le composé du titre « trans » sous forme de solide blanc.
P.f. 98-103°C.

Par traitement de ce composé avec une solution d'acide chlorhydrique dans de l'éthanol on obtient son chlorhydrate.
P.f. 305-308°C.

On obtient le composé du titre « cis » sous forme de solide.
P.f. 46-48°C.

### PREPARATION 4

### 4-benzyloxy-1-((2S)2,3-époxypropoxy)-3-(N-phénylsulfonyl-N-tert-butoxycarbonyl-amino)-benzène

### (i) Acétate de 4-benzyloxy-3-(phénylsulfonyl-amino)-phényle

On agite à la température ambiante pendant une nuit, un mélange de 5,0 g (0,0194 mole) d'acétate de 4-benzyloxy-3-amino-phényle, 3,3 ml (0,0236 mole) de triéthylamine et 3,0 ml (0,0236 mole) de chlorure de benzènesulfonyle dans 150 ml de chlorure de méthylène. On lave ensuite à l'eau on sèche la phase organique et on purifie par chromatographie sur colonne de gel de silice en éluant avec un mélange de cyclohexane/acétate d'éthyle=7/3. On obtient le composé du titre.
P.f. 109-111°C.

### (ii) Acétate de 4-benzyloxy-3-(N-tert-butoxycarbonyl-N-phénylsulfonyl-amino)-phényle

On agite à la température ambiante pendant une nuit, un mélange de 5,4 g (0,0136 mole) du produit de l'étape précédente, 3,6 g (0,0163 mole) de di-*tert*-butyldicarbonate et 0,33 (0,00272 mole) de 4-diméthylaminopyridine dans 100 ml de chlorure de méthylène. On évapore ensuite le solvant et on recristallise le produit du titre ainsi obtenu dans de l'acétate d'éthyle.
P.f. 172-174°C.

### (iii) 4-benzyloxy-3-(N-tert-butoxycarbonyl-N-phénylsulfonyl-amino)-phénol

On agite à la température ambiante pendant 40 minutes, un mélange de 3,8 g (0,0076 mole) du produit de l'étape précédente dans 200 ml de méthanol et 9,1 ml d'une solution 1M de NaOH. On ajoute ensuite de l'acide citrique jusqu'à pH 6 et on évapore le solvant. On reprend le résidu dans du chlorure de méthylène, on lave à l'eau, on sèche la phase organique et on évapore le solvant et on traite le produit dans de l'éther isopropylique. On obtient le composé du titre.
P.f .170-172°C.

### (iv) 4-benzyloxy-1-((2S)2,3-époxypropoxy)-3-(N-phénylsulfonyl-N-tert-butoxycarbonyl-amino)-benzène

On agite au reflux pendant une nuit, un mélange de 2,9 g (0,0063 mole) du produit de l'étape précédente, 2,9 g de carbonate de potassium râpé, 2,0 g (0,0078 mole) de (2S)(+)glycidyl nosylate dans 150 ml d'acétone. On filtre, on évapore le solvant et on purifie par chromatographie sur colonne de gel de silice en éluant avec un mélange de cyclohexane/acétate d'éthyle=8/2. On obtient le composé du titre qu'on recristallise dans de l'acétate d'éthyle.
P.f. 152-154°C.

### PREPARATION 5

### 4-benzyloxy-3-(N-n-butylsulfonyl-N-tert-butoxycarbonyl-amino)-1-((2S)2,3-époxypropoxy)-benzène

En opérant comme décrit dans la Préparation 4 mais en utilisant le chlorure de n-butylsulfonyle au lieu du chlorure de benzènesulfonyle on obtient le composé du titre.
P.f. 88-90°C

### PREPARATION 6

### 4-benzyloxy-3-(N-benzylsulfonyl-N-tert-butoxycarbonyl-amino)-1-((2S)2,3-époxypropoxy)-benzène

En opérant comme décrit dans la Préparation 4 mais en utilisant le benzylsulfonylchlorure au lieu du chlorure de benzènesulfonyle on obtient le composé du titre.
P.f. 123-125°C

### PREPARATION 7

### 4-benzyloxy-1-((2S)2,3-époxypropoxy)-3-(N-méthylsulfonyl-N-benzyl-amino)-benzène

### (i) Acétate de 4-benzyloxy-3-(N-méthylsulfonyl-N-benzyl-amino)-phényle

On agite au reflux pendant 4 heures un mélange de 7,7 g (0,023 mole) d'acétate de 4-benzyloxy-3-(N-méthylsulfonyl-amino)-phényle 4,75 g (0,035 mole) de carbonate de potassium râpé et 3,3 ml (0,0276 mole) de bromure de benzyle dans 150 ml d'acétone anhydre. On filtre, on évapore et on obtient le composé du titre qu'on recristallise dans de l'acétate d'éthyle.
P.f. 143-145°C.

### (ii) 4-benzyloxy-3-(N-benzyl-N-méthylsulfonyl-amino)-phénol

En opérant comme décrit dans la Préparation 4 (iii) mais en utilisant le produit de l'étape précédente, on obtient le composé du titre.
P.f .156-158°C.

### (iii) 4-benzyloxy-1-((2S)2,3-époxypropoxy)-3-(N-benzyl-N-méthylsulfonyl-amino)-benzène

En opérant comme décrit dans la Préparation 4 (iv) mais en utilisant le produit de l'étape précédente, on obtient le composé du titre.
P.f.112-113°C.

### PREPARATION 8

### Trans 4-(4-amino-cyc1ohexyl)-N,N-diéthyl-benzamide

### (i) Trans 4-(4-(N-benzyloxycarbonyl-amino)-cyclohexyl)-benzoate d'éthyle

On agite à la température ambiante pendant 3 heures un mélange de 2,0 g (0,0008 mole) de trans 4-(4-amino-cyclohexyl)-benzoate d'éthyle, 1,25 ml de triéthylamine et 1,26 ml (0,0084 mole) de chloroformiate de benzyle à 95% dans 40 ml de diméthylformamide. On verse le mélange dans de l'eau, on extrait à l'acétate d'éthyle, on sèche et on évapore le solvant. On purifie par chromatographie sur colonne de gel de silice en éluant avec un mélange de cyclohexane/acétate d'éthyle=8/2. On obtient le composé du titre.
P.f. 158-160°C.

### (ii) Acide trans 4-(4-(N-benzyloxycarbonyl-amino)-cyclohexyl)-benzoïque

On hydrolyse l'ester de l'étape précédente avec une solution d'éthanol/tétrahydrofurane en présence de NaOH. On obtient le composé du titre.
P.f. 249-251°C

### (iii) Trans 4-(4-(N-benzyloxycarbonyl-amino)-cyclohexyl)-N,N-diéthyl-benzamide

On chauffe à 40°C pendant 5 heures et après à la température ambiante pendant une nuit, un mélange de 650 mg (1,84 mmole) du produit de l'étape précédente, 814 g (1,84 mmole) de BOP, 0,190 ml (1,84 mmole) de diéthylamine et 0,258 ml (1,84 mmole) de triéthylamine dans 30 ml de chlorure de méthylène. On évapore le solvant, on reprend le résidu dans le d'acétate d'éthyle, on lave avec une solution de bicarbonate de sodium et ensuite avec une solution aqueuse d'acide acétique; on sèche la phase organique et on évapore le solvant. On purifie par chromatographie sur colonne de gel de silice en éluant avec un mélange de cyclohexane/acétate d'éthyle=1/1. On obtient le composé du titre.
P.f. 122-125°C.

### (iv) Trans 4-(4-amino-cyclohexyl)-N,N-diéthyl-benzamide

On agite une solution de 610 mg du produit de l'étape précédente dans 20 ml d'éthanol à la température de 40°C en atmosphère d'hydrogène pendant 7 heures en présence de 70 mg Pd/C à 10%. On filtre le catalyseur et on évapore le solvant sous pression réduite et on traite le produit avec de l'éther éthylique. On obtient le composé du titre.
P.f. 180-182°C.

### PREPARATION 9

### Trans 4-(4-amino-cyclohexyl)-N-butyl-benzamide

En opérant comme décrit dans la Préparation 8 (iii) et (iv) mais en utilisant la n-butylamine au lieu de la diéthylamine, on obtient le composé du titre.
P.f. 108-110°C.

### PREPARATION 10

### Trans (4-N-benzylamino-cyclohexyl)-benzène

On mélange 5,0 g de 4-phénylcyclohexanone (0,028 mole), 3,32 g de benzylamine (0,031 mole), 2,14 g de NaBH₃CN (0,034 mole) (rajouté à 0°C) et 3,75 g d'acide acétique dans 100 ml d'éthanol et on agite pendant une nuit à la température ambiante. On ajoute ensuite une solution de bicarbonate de sodium 1N, on agite à la température ambiante pendant 3 heures, on évapore l'éthanol, on extrait à l'acétate d'éthyle et on évapore le solvant. On dissout le produit dans de l'éthanol et on y ajoute une solution éthanol/HCl 3N et on laisse agiter pendant 3 heures. On porte à pH basique avec une solution de bicarbonate de sodium 1N, on évapore l'éthanol, on extrait avec de l'acétate d'éthyle, on sèche la phase organique et on évapore le solvant. On obtient un mélange d'isomère cis et trans qu'on sépare par chromatographie flash en éluant avec un mélange de chlorure de méthylène/méthanol/NH₄OH=98/2/0,2. On obtient le composé du titre comme solide blanc (R.f. inférieur par rapport à l'isomère cis).

### PREPARATION 11

### Trans 1-(4-N-benzylamino-cyclohexyl)-benzonitrile

En opérant comme décrit dans la Préparation 10 mais en utilisant le 4-(4-cyano-phényl)-cyclohexanone au lieu du 4-phénylcyclohexanone on obtient un mélange d'isomère cis et trans qu'on sépare par chromatographie flash en éluant avec un mélange de chloroforme/méthanol=9/1. On obtient le composé du titre comme solide blanc (R.f. inférieur par rapport à l'isomère cis - sémi-solide).
P.f.117-119°C.

### PREPARATION 12

### Trans 4-(4-N-benzylamino-cyclohexyl)-benzamide

On mélange 1,1 g (3,90 mmole) du produit de la Préparation 11, 1 ml de NaOH à 20% et 1 ml de H₂O₂ à 30%. On agite à la température ambiante pendant 15 minutes puis on ajoute lentement 5 ml de méthanol. On obtient une solution jaune qu'on agite vigoureusement pendant 5 heures. On dilue la solution blanche ainsi obtenue avec 50 ml d'eau et on extrait au chlorure de méthylène. On sèche la phase organique et on évapore le solvant. On traite le produit dans de l'éther diéthylique. On obtient le produit du titre.
P.f. 207-210°C.

### PREPARATION 13

### 4-méthoxy-3-[(N-methylsulfonyl-N-tert-butoxycarbonyl)-amino]-1-((2S)2,3-époxypropoxy)-benzène

En opérant comme décrit dans la Préparation 4(ii), (üi) et (iv) mais en utilisant l'acétate de 4-méthoxy-3-(méthylsulfonyl-amino)-phényle au lieu de l'acétate de 4-benzyloxy-3-(phénylsulfonyl-amino)-phényle, on obtient le composé du titre.
P.f. 133-135°C

### PREPARATION 14

### Trans 1-(4-N-benzylamino-cyclohexyl)-benzoate d'éthyle

En opérant comme décrit dans la Préparation 10 mais en utilisant le 4-(4-ethoxycarbonyl-phényl)-cyclohexanone au lieu du 4-phénylcyclohexanone on obtient un mélange d'isomère cis et trans qu'on sépare par chromatographie flash en éluant avec un mélange de cyclohexane/acétate d'éthyle=7/3. On obtient le composé du titre comme solide blanc (R.f. inférieur par rapport à l'isomère cis, semi-solide).
P.f. 74-76°C.

### PREPARATION 15

### 3-(méthylsulfonyl)-5-[(2S)-oxyranylméthoxy]-1,3-benzoxazol-2(3H)-one

### (i) 5-méthylcarbonyl-1,3-benzoxazol-2(3H)-one

A un mélange de 4,2 g (0,0277 mole) de 2-amino-4-méthylcarbonyl-phénol dans 100 ml de THF, on ajoute, à 0°C, 7,8 ml de triéthylamine et 2,75 g (0,0093 mole) de triphosgène. On agite à la temérature ambiante pendant 1 heure, on verse dans 100 ml d'une solution de HCl 0,5 N, on extrait à l'acétate d'éthyle, on sèche la phase organique et on évapore le solvant. On obtient le composé du titre.
P.f.231-234°C.

### (ii) 3-méthylsulfonyl-5-méthylcarbonyl-1,3-benzoxazol-2(3H)-one

On dissout 1,7 g (0,0096 mole) du produit de l'étape précédente dans 60 ml de chlorure de méthylène anhydre et on y ajoute 1,35 ml de triéthylamine et après, à 0°C, 0,75 ml (0,0096 mole) de chlorure de mésyle sous courant d'azote. On agite à la température ambiante pendant une nuit. On verse dans de l'eau, on sèche la phase organique et on évapore le solvant. On purifie le produit par chromatographie en éluant avec un mélange de cyclohexane/acétate d'éthyle=6/4. On obtient le composé du titre.
P.f. 140-143°C.

### (iii) acétate de 3-méthylsulfonyl-1,3-benzoxazol-2(3H)-one-5-yle

On chauffe au reflux pendant 48 heures un mélange de 3,3 g (0,013 mole) du produit de l'étape précédente et 16,1 g (0,065 mole) d'acide 3-chloro-perbenzoïque (MCPBA) dans 200 ml chlorure de méthylène. On refroidit le mélange, on lave ensuite avec une solution de Na₂S₂O₅ à 20%, après avec une solution saturée en bicarbonate de sodium, avec une solution de iodure de sodium et à l'eau. On sèche la phase organique et on évapore le solvant. On purifie le produit par chromatographie en éluant avec un mélange de cyclohexane/acétate d'éthyle=75/25. On obtient le composé du titre comme solide blanc.
P.f. 159-162°C.

### (iv) 5-hydroxy-3-(méthylsulfonyl)-1,3-benzoxazol-2(3H)-one

On dissout 735 mg (2,71 mmole) du produit de l'étape précédente dans 50 ml d'éthanol et on y ajoute 2,06 g (10,84 mmole) d'acide p-toluènesulfonique. On agite pendant 3 heures et ensuite on dilue avec 100 ml de chlorure de méthylène. On lave avec une solution de bicarbonate de sodium, on sèche la phase organique et on évapore le solvant. On obtient le composé du titre.
P.f. 129-130°C.

### (v) 3-(méthylsulfonyl)-5-[(2S)-oxyranylméthoxy]-1,3-benzoxazol-2(3H)-one

En opérant comme décrit dans la Préparation 4 (iv) mais en utilisant le produit de l'étape précédente au lieu du 4-benzyloxy-3-(N-tert-butoxycarbonyl-N-phénylsulfonyl-amino)-phénol, on obtient le composé du titre.
P.f. 100-102°C.

### PREPARATION 16

### 5-(méthylsulfonyl)-7-[(2S)-oxyranylméthoxy]-2,3,4,5-tétrahydro-1,5-benzoxazepine

### (i) 7-hydroxy-5-(méthylsulfonyl)-2,3,4,5-tétrahydro-1,5-benzoxazepine

On mélange 0,568 g (2,32 mmole) d'acétate de 4-hydroxy-3-méthylsulfonyl-amino-phényle (obtenu par hydrogénation du dérivé benzyloxy correspondant), 0,672 g de carbonate de potassium dans 20 ml de DMF sous courant d'azote et on y ajoute ensuite 0,259 ml de 1,3-dibromopropane. On agite le mélange à la température ambiante pendant une nuit. On ajoute de l'eau et on extrait à l'acétate d'éthyle. On sèche la phase organique et on évapore le solvant. On obtient une huile violette qu'on reprend dans 8 ml de méthanol et 1,88 ml de NaOH 1M. On ajoute donc de l'eau puis de l'acide picrique jusqu'à pH neutre. On évapore le méthanol et on extrait avec de l'acétate d'éthyle. On sèche la phase organique et on évapore le solvant. On purifie le produit par chromatographie en éluant avec un mélange de cyclohexane/acétate d'éthyle=7/3. On obtient le composé du titre.

### (ii) 5-(méthylsulfonyl)-7-[(2S)-oxyranylméthoxy]-2,3,4,5-tétrahydro-1,5-benzoxazepine

En opérant comme décrit dans la Préparation 4(iv) mais en utilisant le produit de l'étape précédente au lieu du produit de l'étape 4 (iii), on obtient le composé du titre comme solide blanc.

### PREPARATION 17

### 4-(2-triméthylsilyl-éthoxyméthoxy)-3-chloro-1-((2S)2,3-époxypropoxy)-benzène

### (i) 3-chloro-4-(2-trimethylsilyl-éthoxyméthoxy)-benzaldéhyde

On dissout 5 g (31,93 mmole) de 5-chloro-4-hydroxybenzaldéhyde dans 300 ml de chlorure de méthylène et on y ajoute à 0°C 6,7 ml (38,3 mmole) de N,N-diisopropyl-N-éthyl-amine et 5,9 ml (33,52 mmole) de 2-trimethylsilyl-éthoxyméthylchlorure (SEMCl). On laisse revenir à la température ambiante et on agite pendant une nuit. On ajoute donc de l'eau et on extrait au chlorure de méthylène. On sèche la phase organique et on évapore le solvant. On obtient le produit du titre qu'on purifie par chromatographie sur colonne de gel de silice en éluant avec un mélange heptane/acétate d'éthyle=95/5 puis 9/1.

### (ii) 3-chloro-4-(2-trimethylsilyl-éthoxyméthoxy)-phénol

On ajoute à un mélange de 7,5 g (26,14 mmole) du produit de l'étape précédente dans 260 ml de chlorure de méthylène à 0°C, 10,6 g d'acide meta-chloroperbenzoique à 70%. On laisse revenir à la température ambiante et on agite pendant une nuit. On ajoute donc une solution de bicarbonate de sodium et on extrait au chlorure de méthylène. On sèche la phase organique et on évapore le solvant. On obtient le produit du titre qu'on purifie par chromatographie sur colonne de gel de silice en éluant avec un mélange cyclohexane/acétate d'éthyle=9/1.

### (iii) 4-(2-triméthylsilyl-éthoxyméthoxy)-3-chloro-1-((2S)2,3-époxypropoxy)-benzène

En opérant comme décrit dans la Préparation 4 (iv) masi en utilisant le produit de l'étape précédente au lieu du produit de l'étape 4 (iii), on obtient le composé du titre comme solide blanc.

### PREPARATION 18

### Trans N-[4-(4-amino-cyclohexyl)-benzoyl]-pyrrolidine

En opérant comme décrit dans la Préparation 8 (iii) et (iv) mais en utilisant la pyrrolidine au lieu de la diéthylamine, on obtient le composé du titre.

### PREPARATION 19

### Cis et Trans 4-[4-(4,4-diméthyl-4,5-dihydro-1,3-oxazol-2-yl)-phényl]-cyclohexanamine

En opérant comme décrit dans la Préparation 3 mais en utilisant la 4-[4-(4,4-diméthyl-4,5-dihydro-1,3-oxazol-2-yl)-phényl]-cyclohexanone, et sans y ajouter l'éthanol saturé en acide chlorhydrique, on obtient le mélange cis et trans du produit du titre dans un rapport d'environ 3/7. Ces isomères sont séparés par HPLC dans les conditions suivantes:
Colonne CHROMOLITH RP 18
Èluant : KH₂PO₄ (0,05 M) pH 3,5/acétonitrile = 80/20
Flux : 1 ml/min.
λ .: 254 nm
TRR1 : 1,00 (isomère trans)
TRR2 : 1,08 (isomère cis)

### PREPARATION 20

### Trans 1-(4-N-benzylamino-cyclohexyl)-4-éthylcarbonyl-benzène

### (i) Trans 1-(4-(N-benzylamino-N-tert-butoxycarbonyl)-cyclohexyl)-benzonitrile

On dissout 380 mg (1,3 mmole) du produit de la Préparation 11 dans 4,5 ml de THF et on y ajoute 343 mg (1,5 mmole) de di-tert-butyl-dicarbonate et 0,220 ml de triéthylamine. On agite à la température ambiante pendant une nuit, on ajoute 40 ml d'eau, on extrait la phase acqueuse avec de l'acétate d'éthyle, on sèche la phase organique et on évapore e solvant. On obtient ainsi le produit du titre.

### (ii) Trans 1-(4-(N-benzylamino-N-tert-butoxycarbonyl)-cyclohexyl)-4-éthylcatbonyl-benzène

On dissout 1,06 g (2,72 mmole) du produit de l'étape précédente dans 40 ml de toluène anhydre et on y ajoute sous courant d'azote 5,4 ml (5,43 mmole) de bromure d'éthylmagnésium (EtMgBr) à la température de 0-5°C. On agite à la température ambiante pendant une nuit, on ajoute 50 ml d'eau, on extrait la phase acqueuse avec de l'acétate d'éthyle, on sèche la phase organique et on évapore le solvant. On obtient ainsi le produit du titre.

### (iii) Trans 1-(4-N-benzylamino-cyclohexyl)-4-éthylcarbonyl-benzène

On agite, à la température ambiante pendant une nuit, un mélange de 1,17 g (2,77 mole) du produit de l'étape précédente et 30,7 ml d'une solution d'acide trifluoroacétique dans chlorure de méthylène à 15%. On ajoute donc de l'acétate d'éthyle et on lave avec une solution de bicarbonate de sodium. On sèche la phase organique et on évapore e solvant. On obtient ainsi le produit du titre.

### PREPARATION 21

### Trans 1-(4-N-benzylamino-cyclohexyl)-benzoate de tert-butyle

On prépare le produit du titre par trans-estérification du produit de la Préparation 14 selon la méthode décrite dans J.O.C., 1997, 62:8240.

### EXEMPLE 1

### Trans 4-[4-((2S)-3-(4-benzyloxy-3-(méthylsulfonyl-amino)-phénoxy)-2-hydroxypropylamino)-cyclohexyl]-benzoate d'éthyle

### Formule (I): A=(a); R₁=Bn ; R=-NHSO₂-Me; n,m=0; R₃=4-COOEt

On chauffe au reflux un mélange de 818 mg de 4-benzyloxy-3-(N-tert-butoxycarbonyl-N-méthylsulfonyl-amino)-1-((2S)2,3-époxypropoxy)-benzène (1,82 mmole) et 450 mg du produit « trans » obtenu selon la Préparation 3 sous forme de base (1,82 mmole) dans 15 ml d'éthanol absolu pendant 16 heures. On refroidit le mélange, on y ajoute 3 ml une solution d'éthanol saturée en acide chlorhydrique et on chauffe à 50 °C pendant 6 heures. On évapore le solvant et on reprend avec un mélange de 50 ml d'une solution saturée en bicarbonate de sodium et 50 ml d'acétate d'éthyle. On lave la phase organique avec une solution aqueuse saturée en NaCl. On sèche la phase organique, on filtre et on évapore le solvant sous pression réduite. On purifie le produit brut par chromatographie sur colonne de gel de silice en éluant avec un mélange chlorure de méthylène/méthanol/NH₄OH=95/510,5. On obtient le composé du titre sous forme de solide blanc.
P.f. 132-134°C.

### EXEMPLE 2

### Chlorhydrate de trans 4-[4-((2S)-2-hydroxy-3-(4-hydroxy-3-(méthylsulfonyl-amino)-phénoxy)-propylamino)-cyclohexyl]-benzoate d'éthyle

### Formule (I): A=(a); R₁=H; R=-NHSO₂-Me; n,m=0; R₃=4-COOEt

On agite une solution de 500 mg du produit de l'Exemple 1 (838 mmole) dans 25 ml d'un mélange éthanol et THF à la température ambiante en atmosphère d'hydrogène pendant 7 heures en présence de 400 mg Pd/C à 10%. On filtre le catalyseur, on évapore le solvant sous pression réduite et on purifie le produit brut par chromatographie sur colonne de gel de silice en éluant avec un mélange chlorure de méthylène/méthanol/NH₄OH=95/5/0,5. On obtient le composé du titre sous forme de base. Par traitement de la base avec une solution d'acide chlorhydrique dans de l'éthanol on obtient son chlorhydrate.
P.f. 183-185°C.

On purifie ultérieurement le produit ainsi obtenu par cristallisation dans de l'isopropanol.
P.f. 188-190°C.

### EXEMPLE 3

### Cis 4-[4-((2S)-2-hydroxy-3-(4-hydroxy-3-(méthylsulfonyl-amino) phénoxy)-propylamino)-cyclohexyl]-benzoate d'éthyle

### Formule (I): A=(a); R₁=H; R=-NHSPO₂-Me; n,m=0; R₃=4-COOEt

### 3a. Cis-4-[4-((2S)-3-(4-benzyloxy-3-(N-méthylsulfonyl-amino)-phénoxy)-2-hydroxy-propylamino)-cyclohexyl]-benzoate d'éthyle

En opérant comme décrit dans l'Exemple 1 mais en utilisant le produit cis de la Préparation 3, on obtient le composé du titre comme solide blanc vitreux.

### 3b. Cis 4-[4-((2S)-2-hydroxy-3-(4-hydroxy-3-(méthylsulfonyl-amino)-phénoxy)-propylamino)-cyclohexyl]-benzoate d'éthyle et son chlorhydrate

En opérant comme décrit dans l'Exemple 2 mais en utilisant le produit de l'étape précédente au lieu du produit de l'Exemple 1, on obtient le composé du titre.
P.f. 135-138°C (chlorhydrate)

### EXEMPLE 4

### Trans 4-[4-((2S)-2-hydroxy-3-(4-hydroxy-3-(phénylsulfonyl-amino)-phénoxy)-propylamino)-cyclohexyl]-benzoate d'éthyle et son chlorhydrate

### Formule (I): A=(a); R₁=H; R=-NHSO₂-Ph; n,m=0; R₃=4-COOEt

### 4a. Trans 4-[4-((2S)-2-hydroxy-3-(4-benzyloxy-3-(phénylsulfonyl-amino)-phénoxy)-propylamino)-cyclohexyl]-benzoate d'éthyle

En opérant comme décrit dans l'Exemple 1 mais en utilisant l'époxyde de la Préparation 4, on obtient le composé du titre.
P.f. 113-115°C.

### 4b. Trans 4-[4-((2S)-2-hydroxy-3-(4-hydroxy-3-(phénylsulfonyl-amino)phénoxy)-propylamino)-cyclohexyl]-benzoate d'éthyle et son chlorhydrate

En opérant comme décrit dans l'Exemple 2 mais en utilisant le produit de l'Exemple 4a au lieu du produit de l'Exemple 1, on obtient le composé du titre.
P.f. 172-174°C (chlorhydrate).

### EXEMPLE 5

### Trans 4-[4-((2S)-2-hydroxy-3-(4-hydroxy-3-(n-butylsulfonyl-amino) phénoxy)-propylamino)-cyclohexyl]-benzoate d'éthyle et son chlorhydrate

### Formule (I): A=(a); R₁=H; R=-NHSO₂-nBu; n,m=0; R₃=4-COOEt

### 5a. Trans 4-[4-((2S)-2-hydroxy-3-(4-benzyloxy-3-(n-butylsulfonyl-amino)-phénoxy)-propylamino)-cyclohexyl]-benzoate d'éthyle

En opérant comme décrit dans l'Exemple 1 mais en utilisant l'époxyde de la Préparation 5, on obtient le composé du titre.
P.f. 108-110°C.

### 5b. Trans 4-[4-((2S)-2-hydroxy-3-(4-hydroxy-3-(n-butylsulfonyl-amino)phénoxy)-propylamino)-cyclohexyl]-benzoate d'éthyle et son chlorhydrate

En opérant comme décrit dans l'Exemple 2 mais en utilisant le produit de l'Exemple 5a au lieu du produit de l'Exemple 1, on obtient le composé du titre.
P.f. 149-151°C (chlorhydrate).

### EXEMPLE 6

### Trans 4-[4-((2S)-2-hydroxy-3-(4-hydroxy-3-(benzylsulfonyl-amino)-phénoxy)-propylainino)-cyclohexyl]-benzoate d'éthyle et son chlorhydrate

### Formule (I): A=(a); R₁=H; R=-NHSO₂-Bn; n,m=0; R₃=4-COOEt

### 6a. Trans 4-[4-((2S)-2-hydroxy-3-(4-benzyloxy-3-(benzylsulfonyl-amino)-phénoxy)-propylamino)-cyclohexyl]-benzoate d'éthyle

En opérant comme décrit dans l'Exemple 1 mais en utilisant l'époxyde de la Préparation 6, on obtient le composé du titre sous forme d'un solide blanc vitreux.

### 6b. Trans 4-[4-((2S)-2-hydroxy-3-(4-hydroxy-3-(benzylsulfonyl-amino)-phénoxy)-propylamino)-cyclohexyl]-benzoate d'éthyle et son chlorhydrate

On dissout 430 mg du produit de l'étape précédente dans 7,5 ml d'acide trifluoroacétique et on chauffe le mélange à 60°C pendant 3 heures. On évapore le solvant sous pression réduite, et on reprend le résidu dans un mélange de bicarbonate de sodium aqueux et acétate d'éthyle. On rajoute 300 mg de carbonate de potassium et on sépare les deux phases. On lave la phase organique avec une solution de chlorure de sodium, on la sèche sur du sulfate de sodium et on évapore le solvant sous pression réduite. On purifie le résidu par flash-chromatographie sur une colonne de gel de silice en éluant par un mélange CH₂Cl₂/MeOH/NH₃=95/5/05. On obtient ainsi le produit du titre. On prépare son chlorhydrate à l'aide d'une solution d'acide chlorhydrique dans de l'acétate d'éthyle.
P.f. 170-172°C (chlorhydrate).

### EXEMPLE 7

### Trans 4-[4-((2S)-2-hydroxy-3-(4-hydroxy-3-(méthylsulfonyl)-phénoxy)-propylamino)-cyclohexyl]-benzoate d'éthyle

### Formule (I): A=(a); R₁=H; R=-SO₂-Me; n,m=0; R₃=4-COOEt

### 7a. Trans 4-[4-((2S)-2-hydroxy-3-(4-benzyloxy-3-(méthylsulfonyl)-phénoxy)-propylamino)-cyclohexyl]-benzoate d'éthyle

En opérant comme décrit dans l'Exemple 1 mais en utilisant le 4-benzyloxy-3-méthylsulfonyl-1-((2S)2,3-époxypropoxy)-benzène (décrit dans WO 99/65895), et sans y ajouter la solution d'acide chlorhydrique en éthanol, on obtient le composé du titre.
P.f. 142-144°C.

### 7b. Trans 4-[4-((2S)-2-hydroxy-3-(4-hydroxy-3-(méthylsulfonyl)-phénoxy)-propylamino)-cyclohexyl]-benzoate d'éthyle

En opérant comme décrit dans l'Exemple 6b mais en utilisant le produit de l'Exemple 7a au lieu du produit de l'Exemple 6a, on obtient le composé du titre.
P.f. 173-175°C.

### EXEMPLE 8

### Trans N-[5-[[(2S)-3-((4-(4-(4,4-diméthyl-4,5-dihydro-1,3-oxazol-2-yl)-phényl)-cyclohexyl)-amino)-2-hydroxypropyl]-oxy]-2-hydroxyphényl]-méthanesulfonamide

### Formule (I): A=(a); R₁=H; R=-NHSO₂-Me; n,m=0; R₃=4-Ox

### 8a. Trans N-[5-[[(2S)-3-((4-(4-(4,4-diméthyl-4,5-dihydro-1,3-oxazol-2-yl)-phényl)-cyclohexyl)-amino)-2-hydroxypropyl]-oxy]-2-benzyloxyphényl]-N-benzyl-N-méthanesulfonamide

En opérant comme décrit dans l'Exemple 1 mais sans y ajouter la solution d'éthanol saturée en HCl, en utilisant l'époxyde de la Préparation 7 et le produit de la Préparation 19, on obtient le composé du titre dépourvu d'isomère cis comme solide vitreux.

### 8b. Trans N-[5-[[(2S)-3-((4-(4-(4,4-diméthyl-4,5-dihydro-1,3-oxazol-2-yl)-phényl)-cyclohexyl)-amino)-2-hydroxypropyl]-oxy]-2-hydroxyphényl]-méthanesulfonamide

En opérant comme décrit dans l'Exemple 2 mais en utilisant le produit de l'Exemple 8a au lieu du produit de l'Exemple 1, on obtient le composé du titre.
P.f. 75-78°C.

### EXEMPLE 9

### Trans 4-[4-((2S)-2-hydroxy-3-(4-hydroxy-3-(méthylsulfonyl-amino)-phénoxy)-propylamino)-cyclohexyl]-N,N-diéthyl-benzamide

### Formule (I): A=(a); R₁=H; R=-NHSO₂-Me; n,m=0; R₃=4-CONEt₂

### 9a. Trans 4-[4-((2S)-2-hydroxy-3-(4-benzyloxy-3-(méthylsulfonyl-amino)-phénoxy)-propylamino)-cyclohexyl]-N,N-diéthyl-benzamide

En opérant comme décrit dans l'Exemple 1 mais en utilisant le produit de la Préparation 8 au lieu du produit de la Préparation 3, on obtient le composé du titre.
P.f. 48-50°C.

### 9b. Trans 4-[4-((2S)-2-hydroxy-3-(4-hydroxy-3-(méthylsulfonyl-amino)-phénoxy)-propylamino)-cyclohexyl]-N,N-diéthyl-benzamide

En opérant comme décrit dans l'Exemple 2 mais en utilisant le produit de l'Exemple 9a au lieu du produit de l'Exemple 1, on obtient le composé du titre.
P.f. 69-72°C.

### EXEMPLE 10

### Trans 4-[4-((2S)-2-hydroxy-3-(4-hydroxy-3-(méthylsulfonyl-amino)-phénoxy)-propylamino)-cyclohexyl]-N-n-butyl-benzamide

### Formule (I): A=(a); R₁=H; R=-NHSO₂-Me; n,m=0; R₃=4-CONHBu

### 10a. Trans 4-[4-((2S)-2-hydroxy-3-(4-benzyloxy-3-(méthylsulfonyl-amino)-phénoxy)-propylamino)-cyclohexyl]-N-n-butyl-benzamide

En opérant comme décrit dans l'Exemple 1 mais en utilisant le produit de la Préparation 9 au lieu du produit de la Préparation 3, on obtient le composé du titre.
P.f. 138-140°C.

### 10b. Trans 4-[4-((2S)-2-hydroxy-3-(4-hydroxy-3-(méthylsulfonyl-amino)-phénoxy)-propylamino)-cyclohexyl]-N-n-butyl-benzamide

En opérant comme décrit dans l'Exemple 2 mais en utilisant le produit de l'Exemple 10a au lieu du produit de l'Exemple 1, on obtient le composé du titre.
P.f. 144-146°C.

### EXEMPLE 11

### Trans 4-[4-((2S)-2-hydroxy-3-(4-hydroxy-3-(méthylsulfonyl-amino)-phénoxy)-propylamino)-cyclohexyl]-benzène

### Formule (I): A=(a); R₁=H; R=-NHSO₂-Me; n,m=0; R₃=H

### 11a. Trans 4-[4-((2S)-2-hydroxy-3-(4-benzyloxy-3-(méthylsulfonyl-amino)-phénoxy)-propyl-(N-benzyl)-amino)-cyclohexyl]-benzène

En opérant comme décrit dans l'Exemple 1 mais en utilisant le produit de la Préparation 10 au lieu du produit de la Préparation 3, et en éluant avec cyclohexane/acétate d'éthyle = 8/2, on obtient le composé du titre.

### 11b. Trans 4-[4-((2S)-2-hydroxy-3-(4-hydrohy-3-(méthylsulfonyl-amino)-phénoxy)-propylamino)-cyclohexyl]-benzène

En opérant comme décrit dans l'Exemple 2 mais en utilisant le produit de l'Exemple 11a au lieu du produit de l'Exemple 1, on obtient le composé du titre.
P.f. 172-175°C.

### EXEMPLE 12

### Acide Trans 4-[4-((2S)-2-hydroxy-3-(4-hydroxy-3-(méthylsulfonyl-amino)-phénoxy)-propylamino)-cyclohexyl]-benzoïque

### Formule (I): A=(a); R₁=H; R=-NHSO₂-Me; n,m=0; R₃=4-COOH

On agite pendant 4 heures une solution de 0.1167 g du produit de l'Exemple 2 (base) (0.33 mmol) dans un mélange de 1,6 mL de méthanol et de 1,6 mL d'une solution aqueuse 1N de soude. On acidifie ensuite le milieu réactionnel acidifié par addition de 1,6 mL d'une solution aqueuse 1N d'acide chlorhydrique, puis on dilue par du méthanol. On obtient le produit du titre (0.08 g, Rdt=5%) sous forme de trifluoroacétate après purification sur HPLC/MS préparative et évaporation des solvants.
Appareils : Deux pompes Shimatzu LC8 couplées à un spectromètre de masse API 100 PE sciex. Un contrôleur SCL-10A. Un injecteur-collecteur de fractions Gilson 215.
Phase stationnaire : Xterra MS C18, 50x30mm, 5 µm

| | |
|---|---|
| Phase mobile | Eluant A : H₂O/MeOH 95/5 + CF₃COOH 0.05% |
| | Eluant B : H₂O/MeOH 5/95 + CF₃COOH 0.05% |
| | Débit : 30mL/min |

Gradient d'élution

| **t (en min)** | %A | %B |
|---|---|---|
| 0 | 90 | 10 |
| 3 | 90 | 10 |
| 15 | 10 | 90 |
| 17 | 10 | 90 |

TR = 8.17 min, [M+H⁺]=479.3.

Le produit purifié a été analysé par HPLC aux conditions suivantes.
Appareils :Deux pompes Shimatzu LC8 couplé à un détecteur UV SPD10-A et à un spectromètre de masse API 100 PE sciex . Un contrôleur SCL-10A. Un injecteur-collecteur de fractions Gilson 215.
Phase stationnaire : Xterra MS C18, 50x4.6mm, 5µm,

| | |
|---|---|
| Phase mobile : | Eluant A : H₂O/MeOH 95/5 + CF₃COOH 0.05% |
| | Eluant B : H₂O/MeOH 5/95 + CF₃COOH 0.05% |
| | Débit : 3mL/min |

Gradient d'élution :

| **t (en min)** | %A | %B |
|---|---|---|
| 0 | 90 | 10 |
| 1 | 90 | 10 |
| 9 | 10 | 90 |
| 10 | 10 | 90 |

TR = 4.79 min, [M+H⁺]=479.3

### EXEMPLE 13

### Trans 4-[4-((2S)-2-hydroxy-3-(4-hydroxy-3-(méthylsulfonyl-amino)-phénoxy)-propylamino)-cyclohexyl]-benzonitrile

### Formule (I): A=(a); R₁=H; R=-NHSO₂-Me; n,m=0; R₃=4-CN

### 13a. Trans 4-[4-((2S)-2-hydroxy-3-(4-benzyloxy-3-(méthylsulfonyl-amino)-phénoxy)-propyl-(N-benzyl)-amino)-cyclohexyl]-benzonitrile

En opérant comme décrit dans l'Exemple 1 mais en utilisant le produit de la Préparation 11 au lieu du produit de la Préparation 3, et en éluant avec chlorure de méthylène/méthanol =9/1, on obtient le composé du titre.

### 13b. Trans 4-[4-((2S)-2-hydroxy-3-(4-hydroxy-3-(méthylsulfonyl-amino)-phénoxy)-propylamino)-cyclohexyl]-benzonitrile

En opérant comme décrit dans l'Exemple 2 mais en utilisant le produit de l'Exemple 13a au lieu du produit de l'Exemple 1, et en utilisant le PdOH₂/C au lieu du Pd/C, on obtient le composé du titre.
¹H NMR (CDCl₃+ D₂O; ppm): 1.13-1.66 (4H; m); 1.77-2.00 (2H; m); 2.00-2.19 (2H; m); 2.35-3.09 (4H; m); 2.89 (3H; s); 3.70-3.93 (2H; m); 3.96-4.16 (1H; m); 6.42 (1H; dd; 9 Hz; 2 Hz); 6.72 (1H; d; 8 Hz); 6.92 (1H; d; 2 Hz); 7.07-7.22 (2H; m); 7.48-7.64 (2H; m).
IR (KBr; cm⁻¹): 3430; ; 2227; 1324; 1151

### EXEMPLE 14

### Trans 4-[4-((2S)-2-hydroxy-3-(4-hydroxy-3-(méthylsulfonyl-amino)-phénoxy)-propylamino)-cyclohexyl]-benzamide

### Formule (I): A=(a); R₁=H; R=-NHSO₂-Me; n,m=0; R₃=4-CONH₂

### 14a. Trans 4-[4-((2S)-2-hydroxy-3-(4-benzyloxy-3-(méthylsulfonyl-amino)-phénoxy)-propyl-(N-benzyl)-amino)-cyclohexyl]-benzamide

En opérant comme décrit dans l'Exemple 1 mais en utilisant le produit de la Préparation 12 au lieu du produit de la Préparation 3, et en éluant avec cyclohexane/acétate d'éthyle = 2/1, on obtient le composé du titre.

### 14b. Trans 4-[4-((2S)-2-hydroxy-3-(4-hydroxy-3-(méthylsulfonyl-amino)-phénoxy)-propylamino)-cyclohexyl]-benzamide

En opérant comme décrit dans l'Exemple 2 mais en utilisant le produit de l'Exemple 14a au lieu du produit de l'Exemple 1, on obtient le composé du titre.
P.f. 79-81°C.

### EXEMPLE 15

### Trans 4-[4-((2S)-2-hydroxy-3-(4-méthoxy-3-(méthylsulfonyl-amino)-phénoxy)-propylamino)-cyclohexyl]-benzoate d'éthyle

### Formule (I): A=(a); R₁=Me; R=-NHSO₂-Me; n,m=0; R₃=4-COOEt

### 15a. Trans 4-[4-((2S)-2-hydroxy-3-(4-méthoxy-3-(méthylsulfonyl-amino)-phénoxy)-propyl-(N-benzyl)-amino)-cyclohexyl]-benzoate d'éthyle

En opérant comme décrit dans l'Exemple 1 mais en utilisant le produit de la Préparation 13, et le produit de la Préparation 14, et en éluant avec cyclohexane/acétate d'éthyle = 1/1, on obtient le composé du titre comme solide vitreux.

### 15b. Trans 4-[4-((2S)-2-hydroxy-3-(4-méthoxy-3-(méthylsulfonyl-amino)-phénoxy)-propylamino)-cyclohexyl]- benzoate d'éthyle

En opérant comme décrit dans l'Exemple 2 mais en utilisant le produit de l'Exemple 15a au lieu du produit de l'Exemple 1, on obtient le composé du titre.
P.f. 144-146°C.

### EXEMPLE 16

### Trans 4-[4-(((2S)-2-hydroxy-3-[(3-(méthylsulfonyl)-2-oxo-2,3-dibydro-1,3-benzoxazol-5-yl]-oxy)propyl)amino)-cyclohexyl]-benzoate d'éthyle

### Formule (I): A=(b); X=CO; R₂=-SO₂Me; n,m=0; R₃=4-COOEt

### 16a. Trans 4-[4-(benzyl-((2S)-2-hydroxy-3-[(3-(méthylsulfonyl)-2-oxo-2,3-dihydro-1,3-benzoxazol-5-yl)-oxy]propyl)amino)-cyclohexyl]-benzoate d'éthyle

En opérant comme décrit dans l'Exemple 1 mais en utilisant l'époxyde de la Préparation 15 et le produit de la Préparation 14 mais sans y ajouter la solution d'éthanol saturée en HCl, et en éluant avec cyclohexane/acétate d'éthyle = 1/1, on obtient le composé du titre comme solide vitreux.

### 16b. Trans 4-[4-(((2S)-2-hydroxy-3-[(3-(méthylsulfonyl)-2-oxo-2,3-dihydro-1,3-benzoxazol-5-yl]-oxy)propyl)amino)-cyclohexyl]-benzoate d'éthyle

En opérant comme décrit dans l'Exemple 2 mais en utilisant le produit de l'Exemple 16a au lieu du produit de l'Exemple 1, on obtient le composé du titre.
P.f. 146-148°C.

### EXEMPLE 17

### Trans 4-[4-((2S)-2-hydroxy-3-((5-méthylsulfony-2,3,4,5-tétrahydro-1,5-benzoxazepin-7-yl)-oxy)-propylamino)-cyclohexyl]-benzoate d'éthyle et son chlorhydrate

### Formule (I): A=(b); X=CH₂CH₂CH₂; R₂=-SO₂Me; n,m=0; R₃=4-COOEt

### 17a. Trans 4-[4-((2S)-2-hydroxy-3-((5-méthylsulfony-2,3,4,5-tétrahydro-1,5-benzoxazepin-7-yl)-oxy)-propyl-(N-benzyl)-amino)-cyclohexyl]-benzoate d'éthyle

En opérant comme décrit dans l'Exemple 1 mais en utilisant l'époxyde de la Préparation 16 et le produit de la Préparation 14, et sans y ajouter la solution d'éthanol saturée en HCl, et en éluant avec chlorure de méthylène/éthanol = 98/2, on obtient le composé du titre.

### 17b. Trans 4-[4-((2S)-2-hydroxy-3-((5-méthylsulfony-2,3,4,5-tétrahydro-1,5-benzoxazepin-7-yl)-oxy)-propylamino)-cyclohexyl]-benzoate d'éthyle et son chlorhydrate

En opérant comme décrit dans l'Exempte 2 mais en utilisant le produit de l'Exemple 17a au lieu du produit de l'Exemple 1, on obtient le composé du titre.
P.f. 170-173°C (chlorhydrate).

### EXEMPLE 18

### Trans 4-[4-((2S)-2-hydroxy-3-(4-hydroxy-3-chloro-phénoxy)-propylamino)-cyclohexyl]-benzoate d'éthyle et son chlorhydrate

### Formule (I): A=(a); R₁=H; R=Cl; n,m=0; R₃=4-COOEt

### 18a. Trans 4-[4-((2S)-2-hydroxy-3-(4-(2-triméthylsilyléthoxyméthoxy)-3-chloro-phénoxy)-propylamino)-cyclohexyl]-benzoate d'éthyle

En opérant comme décrit dans l'Exemple 1 mais en utilisant l'époxyde de la Préparation 17, et sans y ajouter la solution d'acide chlorhydrique, on obtient le composé du titre.

### 18b. Trans 4-[4-((2S)-2-hydroxy-3-(4-hydroxy-3-chloro-phénoxy)-propylamino)-cyclohexyl]-benzoate d'éthyle et son chlorhydrate

On chauffe au reflux pendant une nuit un mélange de 0,34 g (0,588 mmole) du produit de l'étape précédente, 0,46 g (1,76 mmole) de fluorure de tétrabutylammonium et 0,2 ml de hexaméthylphosphoramide dans 5 ml de THF. On évapore le solvant, on reprend à l'acétate d'éthyle et on lave à l'eau, on sèche la phase organique et on évapore le solvant sous pressione réduite. On purifie le produit brut par chromatographie sur colonne de gel de silice en éluant avec un mélange CH₂Cl₂/EtOH=95/5 et après 90/10. On obtient le composé du titre. Par traitement de la base avec une solution d'acide chlorhydrique dans de l'éther éthylique on obtient son chlorhydrate.
P.f. 223-225°C.

### EXEMPLE 19

### Trans N-[4-[4-((2S)-2-hydroxy-3-(4-hydroxy-3-méthylsulfonyl-amino-phénoxy)-propylamino)-cyclohexyl]-benzoyl]-pyrrolidine

### Formule (I): A=(a); R₁=H; R=-NHSO₂-Me; n,m=0; R₃=4-CO-pyrrolidino

### 19a. Trans N-[4-[4-((2S)-2-hydroxy-3-(4-benzyloxy-3-méthylsulfonyl-amino-phénoxy)-propylamino)-cyclohexyl]-benzoyl]-pyrrolidine

En opérant comme décrit dans l'Exemple 1 mais en utilisant le produit de la Préparation 18 au lieu du produit de la Préparation 3, on obtient le composé du titre.

### 19b. Trans N-[4-[4-((2S)-2-hydroxy-3-(4-hydroxy-3-méthylsulfonyl-amino-phénoxy)-propylamino)-cyclohexyl]-benzoyl]-pyrrolidine

En opérant comme décrit dans l'Exemple 2 mais en utilisant le produit de l'étape précédente au lieu du produit de l'Exemple 1, on obtient le composé du titre.
[α]_{D}= -1,6° (c= 0,264, EtOH)

### EXEMPLE 20

### Trans 4-[4-((2S)-2-hydroxy-3-(4-hydroxy-phénoxy)-propylamino)-cyclohexyl]-benzoate d'éthyle

### Formule (I): A=(a); R₁=H; R=H; n,m=0; R₃=4-COOEt

En opérant comme décrit dans l'Exemple 1, mais en utilisant le 4-benzyloxy-1-((2S)2,3-époxypropoxy)-benzène et sans y ajouter la solution d'acide chlorhydrique et après selon l'Exemple 2, on obtient le composé du titre.
P.f. 146°C.

### EXEMPLE 21

### Trans 4-[4-((2S)-2-hydroxy-3-(4-hydroxy-3-(méthylsulfonyl-amino)-phénoxy)-propylamino)-cyclohexyl]-1-éthylcarbonyl-benzène

### Formule (I): A=(a); R₁=H; R=-NHSO₂-Me; n,m=0; R₃=4-COEt

En opérant comme décrit dans l'Exemples 1 mais en utilisant le produit la Préparation 20 au lieu du produit de la Préparation 3 et en éluant avec un mélange cyclohexane/acétate d'éthyle= 7/3, et ensuite selon l'Exemple 2 mais en utilisant le PdOH₂/C au lieu du Pd/C, on obtient le composé du titre sous forme de solide marron clair.
¹H NMR (DMSO-D6 + D₂O 313K; ppm): 1.08 (3H; t; 7 Hz); 1.37-1.68 (4H; m); 1.73-2.00 (2H; m); 2.05-2.29 (2H; m); 2.42-2.70 (2H; m); 2.82-3.21 (4H; m); 2.94 (3H; s); 3.80-3.99 (2H; m); 4.03-4.22 (1H; m); 6.53-6.74 (1H; m); 7.74-6.96 (2H; m); 7.30-7.54 (2H; m); 7.79-8.02 (2H; m).

### EXEMPLE 22

### Trans 4-[4-((2S)-2-hydroxy-3-(4-hydroxy-3-(méthylsulfonyl-amino)-phénoxy)-propylamino)-cyclohexyl]-benzoate de tert-butyle

### Formule (I): A=(a); R₁=H; R=-NHSO₂-Me; n,m=0; R₃=4-COO-tBu

On chauffe au reflux un mélange de 782,4 mg (1,78 mmole) de l'époxyde de la Préparation 7 et 650 mg du produit de la Préparation 21 (1,78 mmole) dans 9 ml d'alcool tert-butylique pendant 16 heures. On évapore le solvant sous pression réduite, on purifie le produit par chromatographie sur colonne de gel de silice en éluant avec chlorure de méthylène/acétate d'éthyle=1/1. On hydrogène à la température ambiante une solution de 880 mg du produit ainsi préparé dans 30 ml de THF, pendant 7 heures en présence de 264 mg Pd/C à 10%. On filtre le catalyseur, on évapore le solvant sous pression réduite et on purifie le produit brut par chromatographie sur colonne de gel de silice en éluant avec du THF. On obtient le composé du titre qu'on purifie par traitement avec du n-pentane.
¹H NMR (DMSO-D6 + D₂O 313K; ppm): 1.10-1.28 (2H; m); 1.38-1.65 (2H; m); 1.53 (9H; s); 1.70-1.1.90 (2H; m); 1.90-2.09 (2H; m); 2.41-2.71 (3H; m); 2.71-2.83 (1H; m); 2.94 (3H; s); 3.72-3.95 (3H; m); 6.61 (1H;dd; 9 Hz; 3 Hz); 6.78 (1H;dd; 9 Hz); 6.82 (1H;dd; 3 Hz); 7.28-7.37 (2H; m); 7.75-7.85 (2H; m).

### EXEMPLE 23

### Trans 4-[4-(((2S)-2-hydroxy-3-[(3-(méthylsulfonyl)-2-oxo-2,3-dihydro-1,3-benzoxazol-5-yl]-oxy)propyl)amino)-cyclohexyl]-benzamide

### Formule (I): A=(b); X=CO; R₂=-SO₂Me; n,m=0; R₃=4-CONH₂

En opérant comme décrit dans l'Exemple 1 mais en utilisant l'époxyde de la Préparation 15 et le produit de la Préparation 12, et sans y ajouter la solution d'éthanol saturée en HCl, et ensuite comme décrit dans l'Exemple 2, on obtient le composé du titre.
¹H NMR (DMSO-D6; 313K; ppm): 1.03-1.29 (2H; m); 1.387-1.63 (2H; m); 1.72-1.90 (2H; m); 1.90-2.11 (2H; m); 2.35-2.48 (1H; m); 2.52-2.84 (3H; m); 3.39 (3H; s); 3.68-3.84 (1H; m); 3.84-4.02 (2H; m); 7.09 (1H; dd; 9 Hz; 3 Hz); 7.25-7.33 (2H; m); 7.38 (1H;d; 3 Hz); 7.41 (1H; d; 9 Hz); 7.73-7.82 (2H; m).
IR (KBr; cm⁻¹): 3381; 3203; 1770; 1657.

## Revendications

1. Composé formule (I) : 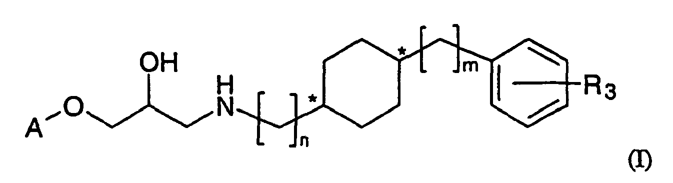 dans laquelle
A est un groupe de formule (a) ou (b) 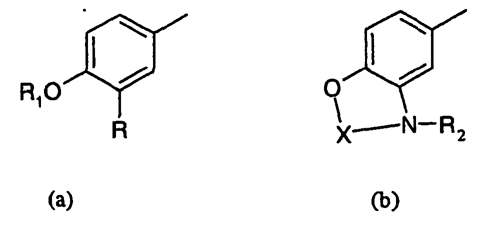
où
R représente un atome d'hydrogène ou d'halogène, un groupe -S(O)_{z}(C₁-C₄)alkyle, un groupe -NHSO₂(C₁-C₄)alkyle, un groupe -SO₂NH(C₁-C₄)alkyle, un groupe -NHSO₂phényl-(C₁-C₄)alkyle ou un groupe -NNSO₂phényle ledit phényle pouvant être substitué par un atome d'halogène, par un groupe (C₁-C₄)alkyle ou par un groupe (C₁-C₄)alcoxy ;
R₁ représente un atome d'hydrogène ou un groupe (C₁-C₄)alkyle, un groupe -CO(C₁-C₄)alkyle, un groupe phényl-(C₁-C₄)alkyle ou un groupe -COphényle, ledit phényle pouvant être substitué par un atome d'halogène ou par un groupe (C₁-C₄)alcoxy;
R₂ est un atome d'hydrogène, un groupe -SO₂(C₁-C₄)alkyle, un groupe -SO₂phényl-(C₁-C₄)alkyle ou un groupe -SO₂phényle,
X complète un cycle de 5 à 8 atomes, ledit cycle étant saturé ou insaturé, pouvant être substitué par un ou deux groupes (C₁-C₄)alkyles et portant un ou deux groupes carbonyles ;
n, m et z sont indépendamment 0, 1 ou 2 ;
R₃ représente un atome d'hydrogène ou d'halogène, un groupe (C₁-C₆)alkyle, un groupe (C₁-C₄)alcoxy, un groupe -COO(C₁-C₄)alkyle, un groupe -CO(C₁-C₄)alkyle, un groupe -NHSO₂(C₁-C₄)alkyle, un groupe -NHSO₂phényl-(C₁-C₄)alkyle, -NO₂, -CN, -CONR₄R₅, -COOH, un groupe 4,5-dihydro-1,3-oxazol-2-yle ou 4,4-diméthyl-4,5-dihydro-1,3-oxazol-2-yle;
R₄ et R₅ représentent indépendamment un atome d'hydrogène, un phényle, un groupe (C₁-C₄)alkyle ou un groupe phényl-(C₁-C₄)alkyle ;
ou bien
R₄et R₅ avec l'atome d'azote auquel ils sont liés, peuvent former un cycle de 5 à 7 atomes au total;
ainsi que ses sels ou solvates.

2. Composé selon la revendication 1 où n et m sont chacun zéro.

3. Composé selon la revendication 1 où R₁ est un atome d'hydrogène.

4. Composé selon la revendication 1 où R est choisi parmi un groupe -NHSO₂(C₁-C₄)alkyle, un groupe -NHSO₂phényl-(C₁-C₄)alkyle ou un groupe -NHSO₂phényle.

5. Composé selon la revendication 1 où R₃ est -CN, -COOH, -COO(C₁-C₄)alkyle ou -CO(C₁-C₄)alkyle.

6. Composé selon la revendication 1 où z est 2.

7. Le 4-[4-((2S)-2-hydroxy-3-(4-hydroxy-3-(méthylsulfonyl-amino)-phénoxy)-propylamino)-cyclohexyl]-benzoate d'éthyle et ses sels ou solvates.

8. Le 4-[4-((2S)-2-hydroxy-3-(4-hydroxy-3-(phénylsulfonyl-amino)-phénoxy)-propylamino)-cyclohexyl]-benzoate d'éthyle et ses sels ou solvates.

9. Composé selon la revendication 1 **caractérisé en ce qu'**il consiste en le:
• Trans 4-[4-((2S)-3-(4-benzyloxy-3-(méthylsulfonyl-amino)-phénoxy)-2-hydroxypropylamino)-cyclohexyl]-benzoate d'éthyle,
• Trans 4-[4-((2S)-2-hydroxy-3-(4-hydroxy-3-(méthylsulfonyl-amino)-phénoxy)-propylamino)-cyclohexyl]-benzoate d'éthyle,
• Cis 4-[4-((2S)-2-hydroxy-3-(4-hydroxy-3-(méthylsulfonyl-amino)phénoxy)-propylamino)-cyclohexyl]-benzoate d'éthyle,
• Trans 4-[4-2S)-2-hydroxy-3-(4-hydroxy-3-(phénylsulfonyl-amino)-phénoxy)-propylamino)-cyclohexyl]-benzoate d'éthyle,
• Trans 4-[4-((2S)-2-hydroxy-3-(4-hydroxy-3-(n-butylsulfonyl-amino)phénoxy)-propylamino)-cyclohexyl]-benzoate d'éthyle,
• Trans 4-[4-((2S)-2-hydroxy-3-(4-hydroxy-3-(benzylsulfonyl-amino)-phénoxy)-propylamino)-cyclohexyl]-benzoate d'éthyle,
• Trans 4-[4-((2S)-2-hydroxy-3-(4-hydroxy-3-(méthylsulfonyl)-phénoxy)-propylamino)-cyclohexyl]-benzoate d'éthyle,
• Trans N-[5-[[(2S)-3-((4-(4-(4,4-diméthyl-4,5-dihydro-1,3-oxazol-2-yl phényl)-cyclohexyl)-amino)-2-hydroxypropyl]-oxy]-2-hydroxyphényl]méthanesulfonamide,
• Trans 4-[4-((2S)-2-hydroxy-3-(4-hydroxy-3-(méthylsulfonyl-amino)-phénoxy)-propylamino)-cyclohexyl]-N, N-diéthyl-benzamide,
• Trans 4-[4-((2S)-2-hydroxy-3-(4-hydroxy-3-(méthylsulfonyl-amino)-phénoxy)-propylamino)-cyclohexyl]-N-n-butyl-benzamide,
• Trans 4-[4-((2S)-2-hydroxy-3-(4-hydroxy-3-(méthylsulfonyl-amino)-phénoxy)-propylamino)-cyclohexyl]-benzène,
• Acide Trans 4-[4-((2S)-2-hydroxy-3-(4-hydroxy-3-(méthylsulfonyl-amino)-phénoxy)-propylamino)-cyclohexyl]-benzoïque,
• Trans 4-[4-((2S)-2-hydroxy-3-(4-hydroxy-3-(méthylsulfonyl-amino)phénoxy)-propylamino)-cyclohexyl]-benzonitrile,
• Trans 4-[4-((2S)-2-hydroxy-3-(4-hydroxy-3-(méthylsulfonyl-amino)-phénoxy)-propylamino)-cyclohexyl]-benzamide,
• Trans 4-[4-((2S)-2-hydroxy-3-(4-méthoxy-3-(méthylsulfonyl-amino)-phénoxy)-propylamino)-cyclohexyl]-benzoate d'éthyle,
• Trans 4-[4-(((2S)-2-hydroxy-3-[(3-(méthylsulfonyl)-2-oxo-2,3-dihydrol, 3-benzoxazol-5-yl]-oxy)propyl)amino)-cyclohexyl]-benzoate d'éthyle,
• Trans 4-[4-((2S)-2-hydroxy-3-5-méthylsulfony-2,3,4,5-tétrahydro-1,5-benzoxazepin-7-yl)-oxy)-propylamino)-cyclohexyl]-benzoate d'éthyle,
• Trans 4-[4-((2S)-2-hydroxy-3-(4-hydroxy-3-chloro-phénoxy)-propylamino)-cyclohexyl]-benzoate d'éthyle
• Trans N-[4-[4-((2S)-2-hydroxy-3-(4-hydroxy-3-méthylsulfonyt-amino-phénoxy)-propylamino)-cyclohexyl]-benzoyl]-pyrrolidine,
• Trans 4-[4-((2S)-2-hydroxy-3-(4-hydroxy-phénoxy)-propylamino)-cyclohexyl]-benzoate d'éthyle
• Trans 4-[4-((2S)-2-hydroxy-3-(4-hydroxy-3-(méthylsulfonyl-amino)-phénoxy)-propylamino)-cyc1ohexyl]-1-éthylcarbonyl-benzène
• Trans 4-[4-((2S)-2-hydroxy-3-(4-hydroxy-3-(méthylsulfonyl-amino)-phénoxy)-propylamino)-cyclohexyl]-benzoate de tert-butyle ou
• Trans 4-[4-(((2S)-2-hydroxy-3-[(3-(méthylsulfonyl)-2-oxo-2,3-dihydrol, 3-benzoxazol-5-yl]-oxy)propyl)amino)-cyclohexyl]-benzamide
ainsi que son sel ou solvate.

10. Procédé pour la préparation d'un composé de la revendication 1 **caractérisé en ce qu'**on
(a) fait réagir le composé de formule (II) : 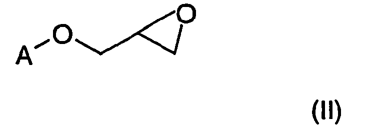
dans laquelle A est tel qu'indiqué dans la revendication 1, avec une amine de formule (III) : 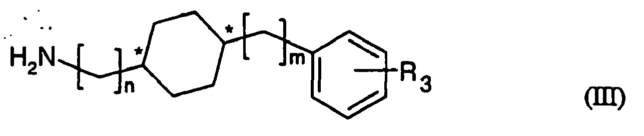 où n, m et R₃ sont tels que définis dans la revendication 1, et éventuellement en on transforme le composé de formule (I) ainsi obtenu en un de ses sels ou solvates.

11. Composition pharmaceutique contenant en tant que principe actif un composé de formule (I) selon les revendications de 1 à 8 ou l'un de ses sels ou solvates pharmaceutiquement acceptables.

12. Utilisation d'un composé de formule (I) selon les revendications de 1 à 8 ou d'un de ses sels ou solvates pharmaceutiquement acceptables pour la préparation de médicaments lipolytiques, anti-obésité, anti-diabétiques, cicatrisants, tocolytiques, ainsi que de médicaments actifs dans la syndrôme du colon irritable (IBD).

13. Médicament comprenant, en tant que principe actif, au moins un composé selon les revendications 1 à 8 ou l'un de ses sels pharmaceutiquement acceptables.

## Claims

1. Compound of formula (I): 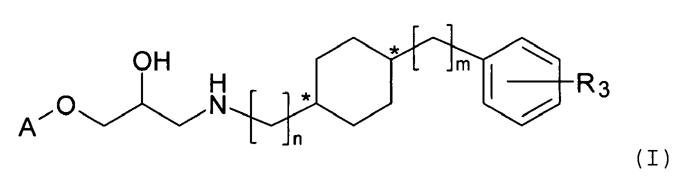 in which
A is a group of formula (a) or (b) 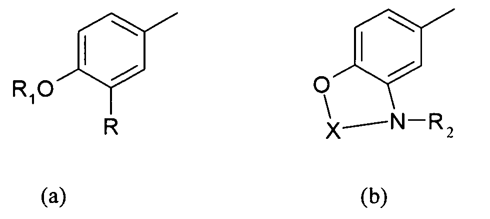
where
R represents a hydrogen or halogen atom, an -S(O)_{z}(C₁-C₄)alkyl group, an -NHSO₂(C₁-C₄)alkyl group, an -SO₂NH(C₁-C₄)alkyl group, an -NHSO₂phenyl- (C₁-C₄)alkyl group or an -NHSO₂phenyl group, said phenyl possibly being substituted with a halogen atom, with a (C₁-C₄)alkyl group or with a (C₁-C₄)alkoxy group;
R₁ represents a hydrogen atom or a (C₁-C₄)alkyl group, a -CO(C₁-C₄)alkyl group, a phenyl-(C₁-C₄)alkyl group or a -COphenyl group, said phenyl possibly being substituted with a halogen atom or with a (C₁-C₄)alkoxy group;
R₂ is a hydrogen atom, an -SO₂(C₁-C₄)alkyl group, an -SO₂phenyl-(C₁-C₄)alkyl group or an -SO₂phenyl group;
X completes a ring of 5 to 8 atoms, said ring being saturated or unsaturated, possibly being substituted with one or two (C₁-C₄)alkyl groups and bearing one or two carbonyl groups;
n, m and z are, independently 0, 1 or 2;
R₃ represents a hydrogen or halogen atom, a (C₁-C₆) alkyl group, a (C₁-C₄) alkoxy group, a -COO(C₁-C₄)alkyl group, a -CO(C₁-C₄)alkyl group, an -NHSO₂(C₁-C₄)alkyl group, an -NHSO₂phenyl-(C₁-C₄) alkyl group, -NO₂, -CN, -CONR₄R₅, -COOH, or a 4,5-dihydro-1,3-oxazol-2-yl or 4,4-dimethyl-4,5-dihydro-1,3-oxazol-2-yl group;
R₄ and R₅ represent, independently, a hydrogen atom, a phenyl, a (C₁-C₄)alkyl group or a phenyl-(C₁-C₄)alkyl group;
or
R₄ and R₅ with the nitrogen atom to which they are attached, may form a ring of 5 to 7 atoms in total;
and the salts or solvates thereof.

2. Compound according to Claim 1, in which n and m are each zero.

3. Compound according to Claim 1, in which R₁ is a hydrogen atom.

4. Compound according to Claim 1, in which R is chosen from an -NHSO₂(C₁-C₄) alkyl group, an -NHSO₂phenyl-(C₁-C₄)alkyl group or an -NHSO₂phenyl group.

5. Compound according to Claim 1, in which R₃ is -CN, -COOH, -COO(C₁-C₄)alkyl or -CO(C₁-C₄)alkyl.

6. Compound according to Claim 1, in which z is 2.

7. Ethyl 4-[4-((2S)-2-hydroxy-3-(4-hydroxy-3-(methylsulphonylamino)phenoxy)propylamino)cyclohexyl] benzoate and the salts or solvates thereof.

8. Ethyl 4-[4-((2S)-2-hydroxy-3-(4-hydroxy-3-(phenylsulphonylamino)phenoxy)propylamino)cyclohexyl] benzoate and the salts or solvates thereof.

9. Compound according to Claim 1, **characterized in that** it consists of:
Ethyl trans-4-[4-((2S)-3-(4-benzyloxy-3-(methylsulphonylamino)phenoxy)-2-hydroxypropylamino)cyclohexyl]benzoate,
Ethyl trans-4-[4-((2S)-2-hydroxy-3-(4-hydroxy-3-(methylsulphonylamino)phenoxy)propylamino)cyclohexyl]benzoate,
Ethyl cis-4-[4-((2S)-2-hydroxy-3-(4-hydroxy-3-(methylsulphonyl-amino)phenoxy)propylamino)cyclohexyl]benzoate,
Ethyl trans-4-[4-((2S)-2-hydroxy-3-(4-hydroxy-3-(phenylsulphonylamino)phenoxy)propylamino)cyclohexyl]benzoate,
Ethyl trans-4-[4-((2S)-2-hydroxy-3-(4-hydroxy-3-(n-butylsulphonylamino)phenoxy)propylamino)cyclohexyl]benzoate,
Ethyl trans-4-[4-((2S)-2-hydroxy-3-(4-hydroxy-3-(benzylsulphonylamino)phenoxy)propylamino)cyclohexyl]benzoate,
Ethyl trans-4-[4-((2S)-2-hydroxy-3-(4-hydroxy-3-(methylsulphonyl)phenoxy)propylamino)cyclohexyl]benzoate,
Trans-N-[5-[[(2S)-3-((4-(4-(4,4-dimethyl-4,5-dihydro-1,3-oxazol-2-ylphenyl)cyclohexyl)amino)-2-hydroxypropyl]oxy]-2-hydroxyphenyl]methanesulphonamide,
Trans-4-[4-((2S)-2-hydroxy-3-(4-hydroxy-3-(methylsulphonylamino)phenoxy)propylamino)cyclohexyl]-N,N-diethylbenzamide,
Trans-4-[4-((2S)-2-hydroxy-3-(4-hydroxy-3-(methylsulphonylamino)phenoxy)propylamino)cyclohexyl]-N-n-butylbenzamide,
Trans-4-[4-((2S)-2-hydroxy-3-(4-hydroxy-3-(methylsulphonylamino)phenoxy)propylamino)cyclohexyl]benzene,
Trans-4-[4-((2S)-2-hydroxy-3-(4-hydroxy-3-(methylsulphonylamino)phenoxy)propylamino)cyclohexyl]benzoic acid,
Trans-4-[4-((2S)-2-hydroxy-3-(4-hydroxy-3-(methylsulphonylamino)phenoxy)propylamino)cyclohexyl]benzonitrile,
Trans-4-[4-((2S)-2-hydroxy-3-(4-hydroxy-3-(methylsulphonylamino)phenoxy)propylamino)cyclohexyl]benzamide,
Ethyl trans-4-[4-((2S)-2-hydroxy-3-(4-methoxy-3-(methylsulphonylamino)phenoxy)propylamino)cyclohexyl]benzoate,
Ethyl trans-4-[4-(((2S)-2-hydroxy-3-[(3-(methylsulphonyl)-2-oxo-2,3-dihydro-1,3-benzoxazol-5-yl]oxy)propyl)amino)cyclohexyl]benzoate,
Ethyl trans-4-[4-((2S)-2-hydroxy-3,5-methylsulphonyl-2,3,4,5-tetrahydro-1,5-benzoxazepin-7-yl)oxy)propylamino)cyclohexyl]benzoate,
Ethyl trans-4-[4-((2S)-2-hydroxy-3-(4-hydroxy-3-chlorophenoxy)propylamino)cyclohexyl]benzoate,
Trans-N-[4-[4-((2S)-2-hydroxy-3-(4-hydroxy-3-methylsulphonylaminophenoxy)propylamino)cyclohexyl]benzoyl]pyrrolidine,
Ethyl trans-4-[4-((2S)-2-hydroxy-3-(4-hydroxyphenoxy)propylamino)cyclohexyl]benzoate,
Trans-4-[4-((2S)-2-hydroxy-3-(4-hydroxy-3-(methylsulphonylamino)phenoxy)propylamino)cyclohexyl]-1-ethylcarbonylbenzene,
tert-Butyl trans-4-[4-((2S)-2-hydroxy-3-(4-hydroxy-3-(methylsulphonylamino)phenoxy)propylamino)cyclohexyl]benzoate or
Trans-4-[4-(((2S)-2-hydroxy-3-[(3-(methylsulphonyl)-2-oxo-2,3-dihydro-1,3-benzoxazol-5-yl]oxy)propyl)amino)cyclohexyl]benzamide
and the salt or solvate thereof.

10. Process for preparing a compound of Claim 1, **characterized in that**
(a) the compound of formula (II): 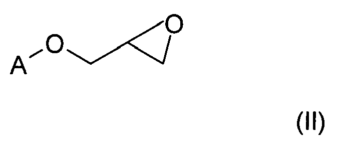
in which A is as indicated in Claim 1, is reacted with an amine of formula (III): 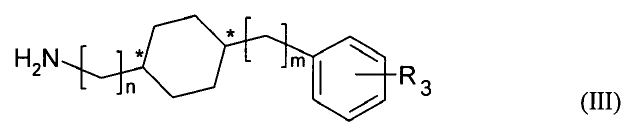 in which n, m and R₃ are as defined in Claim 1 and, optionally, the compound of formula (I) thus obtained is transformed into a salt or solvate thereof.

11. Pharmaceutical composition containing, as an active principle, a compound of formula (I) according to Claims 1 to 9 or a pharmaceutically acceptable salt or solvate thereof.

12. Use of a compound of formula (I) according to Claims 1 to 9, or of a pharmaceutically acceptable salt or solvate thereof, for preparing lipolytic, anti-obesity, anti-diabetic, cicatrizing and to colytic medicinal products, and also medicinal products which are active in irritable bowel disease (IBD).

13. Medicinal product, comprising, as an active principle, at least one compound according to Claims 1 to 9 or a pharmaceutically acceptable salt thereof.

## Patentansprüche

1. Verbindung der Formel (I) : 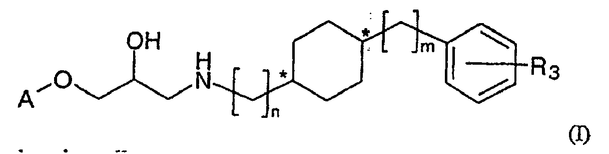 worin
A für eine Gruppe der Formel (a) oder (b) steht: 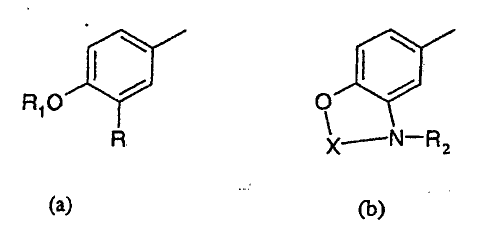
worin
R für ein Wasserstoff- oder Halogenatom oder eine -S(O)_{z}-(C₁-C₄)-Alkylgruppe, eine -NHSO₂-(C₁-C₄)-Alkylgruppe, eine -SO₂NH-(C₁-C₄)-Alkylgruppe, eine -NHSO₂-Phenyl-(C₁-C₄)-alkylgruppe oder eine -NHSO₂-Phenylgruppe steht, wobei das Phenyl durch ein Halogenatom, eine (C₁-C₄)-Alkylgruppe oder eine (C₁-C₄)-Alkoxygruppe substituiert sein kann;
R₁ für ein Wasserstoffatom oder eine (C₁-C₄)-Alkylgruppe, eine -CO-(C₁-C₄)-Alkylgruppe, eine Phenyl- (C₁-C₄)-alkylgruppe oder eine -CO-Phenylgruppe steht, wobei das Phenyl durch ein Halogenatom oder eine (C₁-C₄)-Alkoxygruppe substituiert sein kann;
R₂ für ein Wasserstoffatom oder eine -SO₂-(C₁-C₄)-Alkylgruppe, eine -SO₂-Phenyl-(C₁-C₄)-alkylgruppe oder eine -SO₂-Phenylgruppe steht,
X einen 5- bis 8-atomigen Ring vervollständigt, der gesättigt oder ungesättigt ist, durch eine oder zwei (C₁-C₄)-Alkylgruppen substituiert sein kann und eine oder zwei Carbonylgruppen trägt;
n, m und z unabhängig voneinander für 0, 1 oder 2 stehen;
R₃ für ein Wasserstoff- oder Halogenatom oder eine (C₁-C₆)-Alkylgruppe, eine (C₁-C₄)-Alkoxygruppe, eine -COO-(C₁-C₄)-Alkylgruppe, eine -CO-(C₁-C₄)-Alkylgruppe, eine -NHSO₂-(C₁-C₄)-Alkylgruppe, eine -NHSO₂-Phenyl-(C₁-C₄)-alkylgruppe, -NO₂, -CN, -CONR₄R₅, -COOH, eine 4,5-Dihydro-1,3-oxazol-2-ylgruppe oder eine 4,4-Dimethyl-4,5-dihydro-1,3-oxazol-2-ylgruppe steht;
R₄ und R₅ unabhängig voneinander für ein Wasserstoffatom, Phenyl, eine (C₁-C₄)-Alkylgruppe oder eine Phenyl-(C₁-C₄)-alkylgruppe stehen;
oder
R₄ und R₅ mit dem Stickstoffatom, an das sie gebunden sind, einen Ring mit insgesamt 5 bis 7 Atomen bilden können;
und Salze oder Solvate davon.

2. Verbindung nach Anspruch 1, worin n und m jeweils für Null stehen.

3. Verbindung nach Anspruch 1, worin R₁ für Wasserstoff steht.

4. Verbindung nach Anspruch 1, worin R für eine unter einer -NHSO₂-(C₁-C₄)-Alkylgruppe, einer -NHSO₂-Phenyl-(C₁-C₄)-alkylgruppe oder einer -NHSO₂-Phenylgruppe ausgewählte Gruppe steht.

5. Verbindung nach Anspruch 1, worin R₃ für -CN, -COOH, -COO-(C₁-C₄)-Alkyl oder -CO-(C₁-C₄)-Alkyl steht.

6. Verbindung nach Anspruch 1, worin z für 2 steht.

7. 4-[4-((2S)-2-Hydroxy-3-(4-hydroxy-3-(methylsulfonylamino)phenoxy)propylamino)cyclohexyl]-benzoesäureethylester und Salze oder Solvate davon.

8. 4-[4-((2S)-2-Hydroxy-3-(4-hydroxy-3-(phenylsulfonylamino)phenoxy)propylamino)cyclohexyl]benzoesäureethylester und Salze oder Solvate davon.

9. Verbindung nach Anspruch 1, **dadurch gekennzeichnet, daß** es sich dabei um:
• trans-4-[4-((2S)-3-(4-Benzyloxy-3-(methylsulfonylamino)phenoxy)-2-hydroxypropylamino)cyclohexyl]benzoesäureethylester,
• trans-4-[4-((2S)-2-Hydroxy-3-(4-hydroxy-3-(methylsulfonylamino)phenoxy)propylamino)cyclohexyl]benzoesäureethylester,
• cis-4-[4-((2S)-2-Hydroxy-3-(4-hydroxy-3-(methylsulfonylamino)phenoxy)propylamino)cyclohexyl]benzoesäureethylester,
• trans-4-[4-((2S)-2-Hydroxy-3-(4-hydroxy-3-(phenylsulfonylamino)phenoxy)propylamino)cyclohexyl]benzoesäureethylester,
• trans-4-[4-((2S)-2-Hydroxy-3-(4-hydroxy-3-(n-butylsulfonylamino)phenoxy)propylamino)cyclohexyl]benzoesäureethylester,
• trans-4-[4-((2S)-2-Hydroxy-3-(4-hydroxy-3-(benzylsulfonylamino)phenoxy)propylamino)cyclohexyl]benzoesäureethylester,
• trans-4-[4-((2S)-2-Hydroxy-3-(4-hydroxy-3-(methylsulfonyl)phenoxy)propylamino)cyclohexyl]benzoesäureethylester,
• trans-N-[5-[[(2S)-3-((4-(4-(4,4-Dimethyl-4,5-dihydro-1,3-oxazol-2-ylphenyl)cyclohexyl)amino)-2-hydroxypropyl]oxy]-2-hydroxyphenyl]methansulfonamid,
• trans-4-[4-((2S)-2-Hydroxy-3-(4-hydroxy-3-(methylsulfonylamino)phenoxy)propylamino)cyclohexyl]-N,N-diethylbenzamid
• trans-4-[4-((2S)-2-Hydroxy-3-(4-hydroxy-3-(methylsulfonylamino)phenoxy)propylamino)cyclohexyl]-N-n-butylbenzamid,
• trans-4-[4-((2S)-2-Hydroxy-3-(4-hydroxy-3-(methylsulfonylamino)phenoxy)propylamino)cyclohexyl]benzol,
• trans-4-[4-((2S)-2-Hydroxy-3-(4-hydroxy-3-(methylsulfonylamino)phenoxy)propylamino)cyclohexyl]benzoesäure,
• trans-4-[4-((2S)-2-Hydroxy-3-(4-hydroxy-3-(methylsulfonylamino)phenoxy)propylamino)cyclohexyl]benzonitril,
• trans-4-[4-((2S)-2-Hydroxy-3-(4-hydroxy-3-(methylsulfonylamino)phenoxy)propylamino)cyclohexyl]benzamid,
• trans-4-[4-((2S)-2-Hydroxy-3-(4-methoxy-3-(methylsulfonylamino)phenoxy)propylamino)cyclohexyl]benzoesäureethylester,
• trans-4-[4-(((2S)-2-Hydroxy-3-[(3-(methylsulfonyl)-2-oxo-2,3-dihydro-1,3-benzoxazol-5-yl]-oxy)propyl)amino)cyclohexyl]benzoesäureethylester,
• trans-4-[4-((2S)-2-Hydroxy-3,5-methylsulfonyl-2,3,4,5-tetrahydro-1,5-benzoxazepin-7-yl)oxy)propylamino)cyclohexyl]benzoesäureethylester,
• trans-4-[4-((2S)-2-Hydroxy-3-(4-hydroxy-3-chlorphenoxy)propylamino)cyclohexyl]benzoesäureethylester,
• trans-N-[4-[4-((2S)-2-Hydroxy-3-(4-hydroxy-3-methylsulfonylaminophenoxy)propylamino)cyclohexyl]benzoyl]pyrrolidin
• trans-4-[4-((2S)-2-Hydroxy-3-(4-hydroxyphenoxy)propylamino)cyclohexyl]benzoesäureethylester,
• trans-4-[4-((2S)-2-Hydroxy-3-(4-hydroxy-3-(methylsulfonylamino)phenoxy)propylamino)cyclohexyl]-1-ethylcarbonylbenzol
• trans-4-[4-((2S)-2-Hydroxy-3-(4-hydroxy-3-(methylsulfonylamino)phenoxy)propylamino)cyclohexyl]benzoesäure-tert-butylester oder
• trans-4-[4-(((2S)-2-Hydroxy-3-[(3-(methylsulfonyl)-2-oxo-2,3-dihydro-1,3-benzoxazol-5-yl]oxy)propyl)amino)cyclohexyl]benzamid
handelt, und ein Salz oder Solvat davon.

10. Verfahren zur Herstellung einer Verbindung nach Anspruch 1, **dadurch gekennzeichnet, daß** man
(a) die Verbindung der Formel (II): 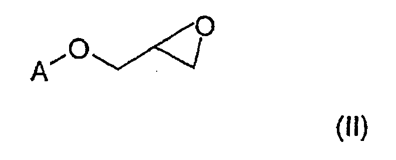
worin A die in Anspruch 1 angegebene Bedeutung besitzt, mit einem Amin der Formel (III): 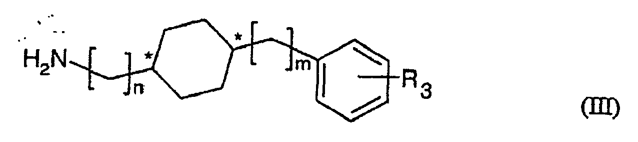 worin n, m und R₃ die in Anspruch 1 angegebene Bedeutung besitzen, umsetzt und gegebenenfalls die so erhaltene Verbindung der Formel (I) in eines ihrer Salze oder Solvate umwandelt.

11. Pharmazeutische Zusammensetzung, enthaltend als Wirkstoff eine Verbindung der Formel (I) nach den Ansprüchen 1 bis 9 oder eines ihrer pharmazeutisch unbedenklichen Salze oder Solvate.

12. Verwendung einer Verbindung der Formel (I) nach den Ansprüchen 1 bis 9 oder eines ihrer pharmazeutisch unbedenklichen Salze oder Solvate zur Herstellung von lipolytisch wirkenden Arzneimitteln, Antiadipositas-Arzneimitteln, antidiabetisch wirkenden Arzneimitteln, vernarbend wirkenden Arzneimitteln, tocolytisch wirkenden Arzneimitteln und bei Reizkolon (IBD) wirksamen Arzneimitteln.

13. Arzneimittel, enthaltend als Wirkstoff mindestens eine Verbindung nach den Ansprüchen 1 bis 9 oder eines ihrer pharmazeutisch unbedenklichen Salze.
